# EUROPEAN PATENT APPLICATION

(11) **EP 1 491 233 A1**
(43) Date of publication of application: **29.12.2004**
(21) Application number: 02783697.2
(22) Date of filing: 29.11.2002
(51) Int. Cl.: A61M 37/00

(54) **MEDICAL TREATMENT SYSTEM AND PRODUCTION METHOD THEREFOR**

(30) Priority: 26.03.2002 JP 2002086423
(71) Applicant: Juridical Foundation Osaka Industrial Promotion Organization, Osaka-shi, osaka 540-0029 (JP)
(72) Inventor: AOYAGI, Seiji, Suita-shi, Osaka 564-0073 (JP); ISONO, Yoshitada, Kusatsu-shi, Shiga 525-0059 (JP); HASHIGUCHI, Gen, Takamatsu-shi, Kagawa 761-8063 (JP)
(74) Representative: VOSSIUS & PARTNER
(86) International application number: PCT/JP2002/012490
(87) International publication number: WO 2003/080174

(57) **Abstract**

The present invention has a purpose to provide a non-invasive drug delivery system made of biodegradable material slowly releasing a medicament for a prolonged period in a stable manner while embedded within a portion of a body where a flow of blood and/or lymph is rapid, and a manufacturing process thereof. The drug delivery system according to the present invention includes a tank member of biodegradable material having a chamber capable of holding a medicament. Also, it has at least one anchor member of biodegradable material extending from the tank member. The anchor member is tapered toward a tip thereof, and has at least one protruding portion extending therefrom.

## Description

### BACKGROUND OF THE INVENTION

### 1) Technical field of the Invention

The present invention relates to a medical device and a manufacturing process thereof. In particular, the present invention relates to a non-invasive drug delivery system (DDS) made of biodegradable material slowly releasing a medicament for a prolonged period in a stable manner while embedded within a body portion where a flow of blood and/or lymph is rapid, and the manufacturing process thereof.

### 2) Description of Related Arts

When a patient orally doses a medicament, most of the dosed medicament is generally decomposed in his or her digestive system and/or liver so that the medicinal action of the medicament is lost. Therefore, in practice, expecting most of the dosed medicament being decomposed, much more amount of the medicament than those actually necessary for treatment is orally administered. However, a medicament typically has an adverse effect, for example, an anti-cancer drug is extremely harmful to the normal portions of the patient's body. Thus, several researches for drug delivery systems capable of delivering desired amount of the medicament to the targeted portion of the body have been developed.

One example of the most promising drug delivery systems is a liposome, which is a spherical closed microcapsule of a phospholipid bi-layer encapsulating the medicament. When the liposome collapses due to activation of a complement system, the encapsulated medicament is released out of the liposome. However, the activation mechanism of the complement system has not yet been revealed thoroughly, thus, the liposome is still on the way to be investigated for an effective drug delivery system.

Meantime, our recent innovation in a technical field of a regenerative medicine is remarkable, many excellent researches have been reported. A refining technology of regenerative cells and/or factors required for the regenerative medicine of a blood vessel, bone, and cornea have already been established. For example, in order to regenerate the blood vessel, the regenerative cells and/or factors have to constantly be supplied for a prolonged time period to the local point of the vascular wall. Also, in order to regenerate the fractured bone, the medicament (the regenerative cells and/or factors) should stably be applied to the portion of the broken bone for an extended time until regenerated. Further, the regenerative cells and/or factors have to be released continuously and locally to the desired portion for a long time.

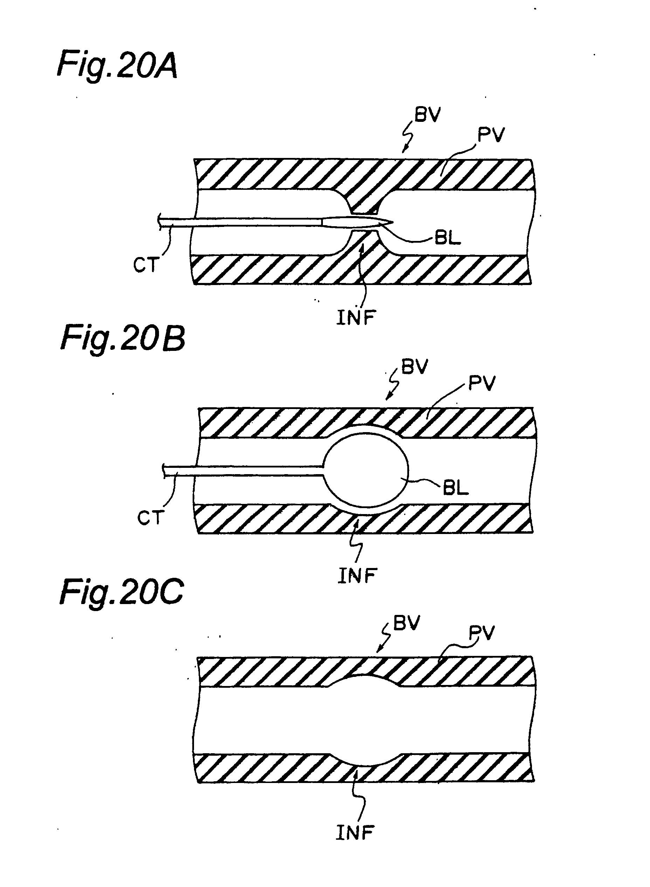

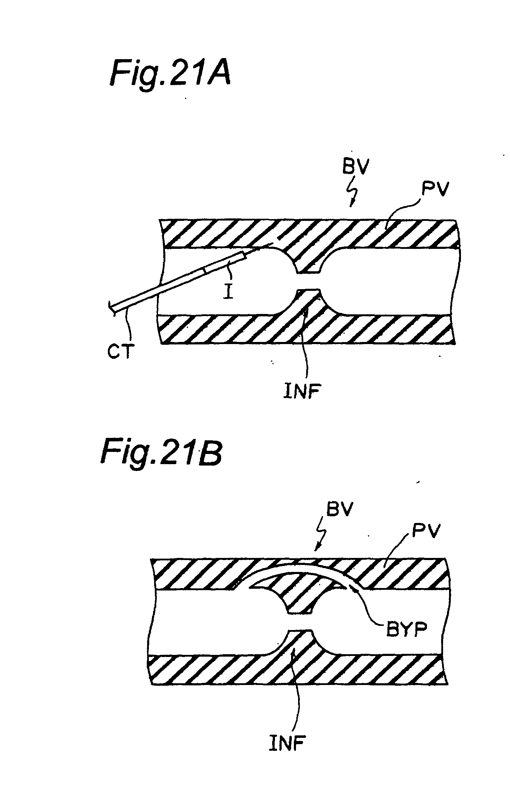

Referring to Figs. 20A-20C and 21A-21B, one example of conventional treatments for a cardiovascular disease with a blockage of a coronary vessel will be described herein. In a typical treatment, firstly, a balloon catheter **CT** having a tip attached with a balloon **BL** is inserted and placed at an infarction **INF** of the coronary vessel **BV**. Then, the balloon **BL** is blown up so that the perivascular tissue **PV** including the narrowed coronary vessel **BV** is expanded, thereby to realize normal circulation of the patient's blood. However, some time after the treatment, the perivascular tissue **PV** is likely to narrow again, thus the cardiovascular disease quite often relapses. When it is difficult to completely cure the cardiovascular disease with this treatment, a coronary bypass surgery is operated. Such a surgical operation is invasive, so that the burden to the patient is much greater than the case taking balloon catheter embolectomy.

To avoid the invasive surgical operation, there has been proposed another approach as illustrated in Fig. 21A, to form a bypassing blood vessel **BYP** for complementing the infarct vessel by using an injection **I** secured on the tip of the catheter **CT** to forward the above-mentioned regenerative cells and/or factors to the blood vessel wall **PV** adjacent the infarct portion **INF** of the blood vessel **BV**.

However, in practice, since the blood vessel wall **PV** is not easily viewed and keeps moving in response to the heart beat, it is impossible to continuously injecting the regenerative cells and/or factors with the injection **I** at the proper position and into the appropriate depth of the blood vessel wall **PV**. In other words, the injection **I** may penetrate deeply enough to reach inside the heart, and also the shallow penetration of the injection I may lose the medicament running from the blood vessel wall **PV** due to the rapid flow of the blood, immediately after the injection **I** is released. Thus, in case where the flow or circulation rate of the blood is high at the local point to be treated for regeneration, the regenerative cells and/or factors cannot be stayed within the blood vessel wall **PV**, contributing no action on the regeneration of the blood vessel.

### SUMMARY OF THE INVENTION

Therefore, the present invention is to address the aforementioned drawbacks, and one of the purposes thereof is to provide a non-invasive drug delivery system made of biodegradable material slowly releasing a medicament for a prolonged period in a stable manner while embedded within a portion of a body where a flow of blood and/or lymph is rapid, and a manufacturing process thereof.

The first aspect of the present invention is to provide a drug delivery system, which includes a tank member of biodegradable material having a chamber capable of holding a medicament, and at least one anchor member of biodegradable material extending from the tank member. The anchor member is tapered toward a tip thereof, and has at least one protruding portion extending therefrom. Therefore, according to the drug delivery system, the sharp tip thereof facilitates easy penetration into the tissue. Also, forming with biodegradable material such as poly-lactic acid and providing the anchor member with the protruding portions allow the drug delivery system to be placed within a body portion where a flow of blood and/or lymph is rapid, providing no harm to the body. In addition, as poly-lactic acid is slowly dissolves, it gently release the medicament held in the tank member in small doses for a predetermined dosing period. This achieves a safer treatment with less burden for a patient instead of the conventional invasive surgery operation.

The second aspect of the present invention is to provide a drug delivery system, which includes a plurality of tank members of biodegradable material, and each of the tank members has a chamber capable of holding a medicament. It also includes a connector member of biodegradable material connecting adjacent tank members, a cap member arranged on the connector member for hermetically sealing each of the tank members, and at least one anchor member of biodegradable material extending from the tank member. The anchor member is tapered toward a tip thereof, and has at least one protruding portion extending therefrom. Therefore, according to the drug delivery system, the sharp tip thereof facilitates easy penetration into the tissue. Also, forming with biodegradable material such as poly-lactic acid and providing the anchor member with the protruding portions allow the drug delivery system to be placed within a body portion where a flow of blood and/or lymph is rapid, providing no harm to the body. In addition, as poly-lactic acid is slowly dissolves, it gently release the medicament held in the tank member in small doses for a predetermined dosing period. Furthermore, a plurality of tank members allows the same or different kind of medicaments to release at different timings.

The third aspect of the present invention is to provide a drug delivery system, which includes an anchor member of biodegradable material having a chamber capable of holding a medicament. The anchor member is tapered toward a tip thereof, and has at least one protruding portion extending therefrom. Thus, the drug delivery system can readily be penetrated into the tissue and placed within the body portion having rapid flow of blood or body fluid, thereby to gently release the medicament held therein in small doses for a predetermined dosing period.

The fourth aspect of the present invention is to provide a drug delivery system, which includes a tank member of biodegradable material containing a medicament therein, and at least one anchor member of biodegradable material extending from the tank member. The anchor member is tapered toward a tip thereof, and has at least one protruding portion extending therefrom. Thus, the drug delivery system can gently release the medicament contained in the biodegradable material such as poly-lactic acid in small doses.

The fifth aspect of the present invention is to provide a drug delivery system, which includes an anchor member of biodegradable material containing a medicament. The anchor member is tapered toward a tip thereof, and has at least one protruding portion extending therefrom. Thus, the drug delivery system can gently release the medicament contained in the biodegradable material such as poly-lactic acid in small doses.

The sixth aspect of the present invention is to provide a drug delivery system, which includes an anchor member of biodegradable material having a tip tapered at one end in a longitudinal direction, and a mass of a medicament attached at the other end. The anchor member has at least one protruding portion extending therefrom. Thus, according to the drug delivery system, the mass of a medicament attached at the other end can be placed at the treatment portion.

The seventh aspect of the present invention is to provide a drug delivery system, which includes an anchor member of biodegradable material having a chamber capable of sealing a medicament injected therein. The anchor member has both ends tapered in a longitudinal direction, and has at least one protruding portion extending therefrom. Thus, the drug delivery system can gently release the medicament stored in the chamber in small doses for a predetermined dosing period.

Preferably, the protruding portion of the anchor member has an outline of a substantial quadrangular pyramid. The protruding portion of a substantial quadrangular pyramid can easily be formed, for example by wet etching the silicon substrate with potassium hydroxide.

Preferably, the protruding portion extends towards a direction inclined to a longitudinal direction towards the tip at an obtuse angle. The protruding portion can easily be formed, for example by ion-reactive etching with sulfur hexafluoride.

Also, it is preferable that the biodegradable material is poly-lactic acid, glue, starch, protein, or glucose.

Preferably, the anchor member has a channel in fluid communication with the chamber of the tank member.

Also, the drug delivery system further includes a plurality of the anchor members extending from the tank member towards different directions.

Also, the drug delivery system further includes a plurality of the anchor members extending from the tank member towards same directions.

Preferably, the tip of the anchor member is tapered as viewing in top plan and cross sectional views.

The eighth aspect of the present invention is to provide a manufacturing process of a drug delivery system, which includes forming semiconductor oxide layers on first and second semiconductor substrates, etching the semiconductor oxide layer on the first semiconductor substrate in a tank region and a plurality of circle regions discretely arranged so as to form a mask of the semiconductor oxide layer, wet etching the first semiconductor substrate with use of the mask of the semiconductor oxide layer, forming a semiconductor oxide layer on the first semiconductor substrate exposed by the wet etching, forming first and second thin layers of poly-lactic acid on the semiconductor oxide layers of the first and second semiconductor substrates, respectively, laminating the first and second semiconductor substrates so that the first and second thin layers of poly-lactic acid are faced to each other, etching the first and second semiconductor substrate, while leaving the semiconductor oxide layers of the first and second semiconductor substrates; and etching the semiconductor oxide layers of the first and second semiconductor substrates, while leaving the first and second thin layers of poly-lactic acid. This allows a mass production of the drug delivery system having the desired dimension and precisely formed shape by means of the micro-machine technology at reasonable cost.

The ninth aspect of the present invention is to provide a manufacturing process of a drug delivery system, which includes forming a semiconductor oxide layer on a semiconductor substrate, etching the semiconductor oxide layer on the semiconductor substrate in a tank region and a plurality of circle regions discretely arranged, except a bridge region extending therethrough so as to form a first mask of the semiconductor oxide layer, wet etching the semiconductor substrate with use of the first mask of the semiconductor oxide layer, forming a semiconductor oxide layer on the semiconductor substrate exposed by the wet etching ,forming a thin layer of poly-lactic acid on the semiconductor oxide layer, forming a thin layer of a given material on the thin layer of poly-lactic acid, etching the thin layer of the given material in a predetermined region so as to form a second mask of the given material, etching the thin layer of poly-lactic acid with use of the second mask of the given material, etching the semiconductor oxide layer with use of the second mask of the given material, etching the semiconductor substrate, while leaving the semiconductor oxide layer, etching the thin layer of the given material, while leaving the thin layer of poly-lactic acid; and etching the semiconductor oxide layer, while leaving the thin layer of poly-lactic acid.

The tenth aspect of the present invention is to provide a manufacturing process of a drug delivery system, which includes forming semiconductor oxide layers on first and second semiconductor substrates, etching the semiconductor oxide layer on the first semiconductor substrate in a tank region and an anchor region so as to form a mask of the semiconductor oxide layer, ion-reactive etching the first semiconductor substrate with use of the mask of the semiconductor oxide layer, forming a semiconductor oxide layer on the first semiconductor substrate exposed by the ion-reactive etching, forming first and second thin layers of poly-lactic acid on the semiconductor oxide layers of the first and second semiconductor substrates, respectively, laminating the first and second semiconductor substrates so that the first and second thin layers of poly-lactic acid are faced to each other, etching the first and second semiconductor substrate, while leaving the semiconductor oxide layers of the first and second semiconductor substrates, and etching the semiconductor oxide layers of the first and second semiconductor substrates, while leaving the first and second thin layers of poly-lactic acid.

The eleventh aspect of the present invention is to provide a manufacturing process of a drug delivery system, which includes forming a semiconductor oxide layer on a semiconductor substrate, etching the semiconductor oxide layer on the semiconductor substrate in a tank region and an anchor region so as to form a mask of the semiconductor oxide layer, ion-reactive etching the semiconductor substrate with use of the mask of the semiconductor oxide layer so as to form a recess on the semiconductor substrate in the tank region and the anchor region, filling up the recess with a given melted material and curing the material so as to form a molding die of the given material, forming a thin layer of poly-lactic acid encompassing the molding die, forming an opening on the thin layer of poly-lactic acid to expose a portion of the molding die, and etching the molding die of the given material, while leaving the thin layer of poly-lactic acid.

The twelfth aspect of the present invention is to provide a manufacturing process of a drug delivery system, which includes forming a semiconductor oxide layer on a semiconductor substrate, etching the semiconductor oxide layer on the semiconductor substrate in an anchor region and a peripheral portion of a tank region so as to form a first mask of the semiconductor oxide layer, ion-reactive etching the semiconductor substrate with use of the first mask of the semiconductor oxide layer so as to form a recess in the anchor region and the peripheral portion of the tank region, filling up the recess with a melted poly-lactic acid so as to form a thin layer of poly-lactic acid, forming a thin layer of a given material on the thin layer of poly-lactic acid, etching the thin layer of the given material in a predetermined region so as to form a second mask of the given material, etching the thin layer of poly-lactic acid with use of the second mask of the given material, etching the semiconductor oxide layer with use of the second mask of the given material, etching the semiconductor substrate, while leaving the semiconductor oxide layer, etching the second mask of the given material, while leaving the thin layer of poly-lactic acid, etching the semiconductor oxide layer, while leaving the thin layer of poly-lactic acid so as to form a structure of poly-lactic acid that includes an opening in a region corresponding to the peripheral portion of the tank region, and covering the opening of the structure of poly-lactic acid by a thin layer of poly-lactic acid.

The thirteenth aspect of the present invention is to provide a manufacturing process of a drug delivery system, which includes forming a tank member of poly-lactic acid having a chamber capable of holding a medicament, forming an anchor member of poly-lactic acid tapered toward to a tip thereof, and the anchor member having at least one protruding portion, and connecting the anchor member with the tank member.

The fourteenth aspect of the present invention is to provide a manufacturing process of a drug delivery system, which includes forming first and second recesses on first and second semiconductor substrates, respectively, filling up the first and second recesses with a given material and curing the material, etching the first and second semiconductor substrates, while leaving the semiconductor oxide layer so as to form first and second molding dice of the given material, filling up a die recess of the first molding die with melted poly-lactic acid, inserting the second molding die into the die recess of the first molding die, etching first and second molding dice of the given material, while leaving poly-lactic acid therebetween so as to form a plurality of tank members, and attaching an anchor member to at least one of the tank members.

The fifteenth aspect of the present invention is to provide a manufacturing process of a drug delivery system, which includes forming first and second semiconductor oxide layers on first and second semiconductor substrates, respectively, etching the first semiconductor oxide layer on the first semiconductor substrate to form a mask of the first semiconductor oxide layer, wet etching the first semiconductor substrate with use of the mask of the first semiconductor oxide layer, forming a semiconductor oxide on the first semiconductor substrate exposed by the wet etching, forming first and second thin layers of poly-lactic acid on the semiconductor oxide layers of the first and second semiconductor substrates, respectively, laminating the first and second semiconductor substrates so that the first and second thin layers of poly-lactic acid are faced to each other, etching the first and second semiconductor substrate, while leaving the semiconductor oxide layers of the first and second semiconductor substrates, and etching the semiconductor oxide layers of the first and second semiconductor substrates, while leaving the first and second thin layers of poly-lactic acid.

The sixteenth aspect of the present invention is to provide a manufacturing process of a drug delivery system, which includes forming a semiconductor oxide layer on a semiconductor substrate, etching the semiconductor oxide layer on the semiconductor substrate in a predetermined mask region so as to form a mask of the semiconductor oxide layer, wet etching the semiconductor substrate with use of the mask of the semiconductor oxide layer so as to form a recess in the predetermined region, filling up the recess with a melted give material and curing the material so as to form a molding die of the given material, forming a thin layer of poly-lactic acid encompassing the molding die, forming an opening on the thin layer of poly-lactic acid to expose a portion of the molding die, and etching the molding die of the given material, while leaving the thin layer of poly-lactic acid.

Preferably, the mask region is defined by sides inclined to a <100> orientation of the semiconductor substrate at an angle of substantially (Π/2 - arctan(√2)).

Preferably, the given material is aluminum.

### BRIEF DESCRIPTION OF THE DRAWINGS

Figs. 1A-1C are perspective view, top plan view, and side view, respectively, of a drug delivery system of the first embodiment according to the present invention.
Figs. 2A-2B are cross sectional views illustrating a bypassing blood vessel formed by means of the drug delivery system of the first embodiment.
Figs. 3A-3H illustrate a manufacturing process of the drug delivery system of the first embodiment, and Figs. 3A, 3D, and 3G are top plan views of a silicon substrate, and Figs. 3B, 3C, 3E, 3F, and 3H are cross sectional views taken along a line IIIB-IIIB of Fig. 3A.
Figs. 4A-4G illustrate a manufacturing process of the drug delivery system of the first embodiment, and all of them are cross sectional views taken along a line IIIB-IIIB of Fig. 3A.
Figs. 5A-5E illustrate an alternative manufacturing process of the drug delivery system of the first embodiment, and Figs. 5A and 5C are top plan views of the silicon substrate, and Figs. 5B, 5D, and 5E are cross sectional views taken along a line VB-VB of Fig. 5A.
Figs. 6A-6F illustrate a further alternative manufacturing process of the drug delivery system of the first embodiment, and Fig. 6A is a top plan view of the silicon substrate, and Figs. 6B-6F are cross sectional views taken along a line VIB-VIB of Fig. 6A.
Figs. 7A-7C are a perspective view, top plan view, and side view, respectively, of a drug delivery system of the second embodiment according to the present invention.
Figs. 8A-8G illustrate a manufacturing process of the drug delivery system of the second embodiment, and Figs. 8A and 8D are top plan views of the silicon substrate, and Figs. 8B, 8C, 8E to 8G are cross sectional views taken along a line VIIIB-VIIIB of Fig. 8A.
Figs. 9A-9G illustrate a manufacturing process of the drug delivery system of the second embodiment, and all of them are cross sectional views taken along a line VIIIB-VIIIB of Fig. 8A.
Figs. 10A-10E illustrate an alternative manufacturing process of the drug delivery system of the second embodiment, and all of them are cross sectional views taken along a line VIIIB-VIIIB of Fig. 8A.
Figs. 11A-11G illustrate a manufacturing process of the drug delivery system of the third embodiment, and Figs. 11A and 11D are top plan views of the silicon substrate, and Figs. 8B, 8C, 8E to 8G are cross sectional views taken along a line XIB-XIB of Fig. 11A.
Figs. 12A-12H illustrate a manufacturing process of the drug delivery system of the third embodiment, and Figs. 12C and 12G are top plan views of a pattern of an aluminum layer and a poly-lactic acid layer, respectively, and Figs. 12A, 12B, 12D to 12F, and 12G are cross sectional views taken along a line XIB-XIB of Fig. 11A.
Figs. 13A-13C are perspective view, top plan view, and side view, respectively, of a drug delivery system of the fourth embodiment according to the present invention.
Figs. 14A-14D illustrate several modifications of the drug delivery system of the fourth embodiment.
Fig. 15A is a perspective view of a drug delivery system of the fifth embodiment according to the present invention, and Fig. 15B is a cross sectional view taken along a line XVB-XVB of Fig. 15A.
Figs. 16A-16F illustrate a manufacturing process of the drug delivery system of the fifth embodiment, and all of them are cross sectional views taken along a line XVB-XVB of Fig. 15A.
Figs. 17A-17C illustrate a manufacturing process of the drug delivery system of the fifth embodiment, and all of them are cross sectional views taken along a line XVB-XVB of Fig. 15A.
Figs. 18A-18C are perspective view, top plan view, and side view, respectively, of a drug delivery system of the sixth embodiment according to the present invention, and Fig. 18D is a cross sectional view taken along a line XVIIID-XVIIID of Fig. 18B.
Figs. 19A-19G illustrate a manufacturing process of the drug delivery system of the sixth embodiment, and Figs. 19B, 19D, 19E and 19G are cross sectional views taken along a line XIXB-XIXB of Fig. 19A.
Figs. 20A-20C illustrate a conventional approach for expanding an infarction of a blood vessel with a balloon catheter.
Figs. 21A and 21B illustrate another conventional approach for expanding an infarction of a blood vessel by injecting the regenerative cells and/or factors by means of the injection.

### DETAILED DESCRIPTION OF THE PREFERRED EMBODIMENTS

Referring to the attached drawings, the details of embodiments of a drug delivery system (DDS) according to the present invention will be described hereinafter. In those descriptions, although the terminology indicating the directions (for example, "upper" and "lower") are conveniently used just for clear understandings, it should not be interpreted that those terminology limit the scope of the present invention.

### «Drug Delivery System of First Embodiment»

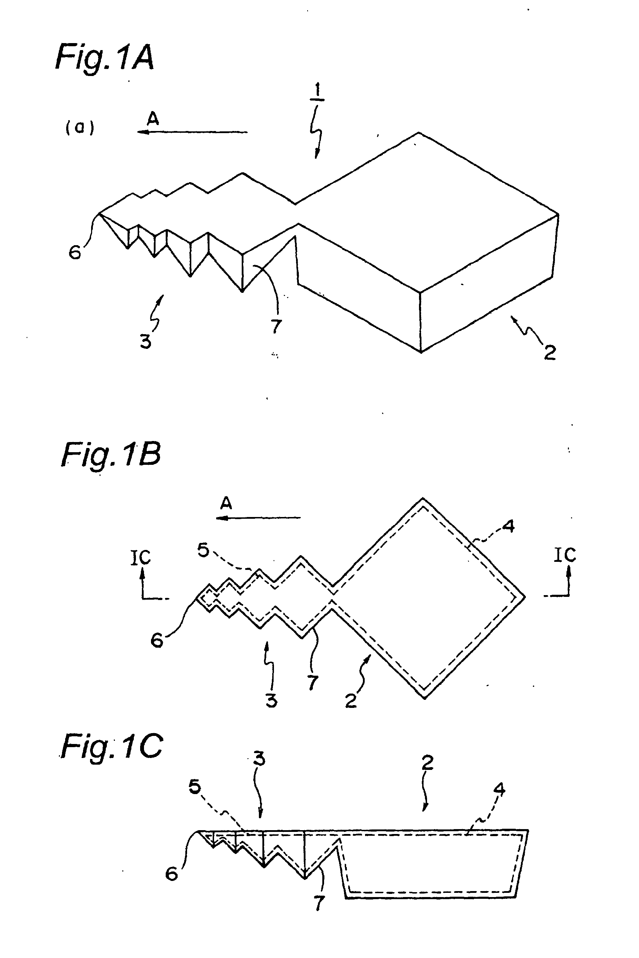

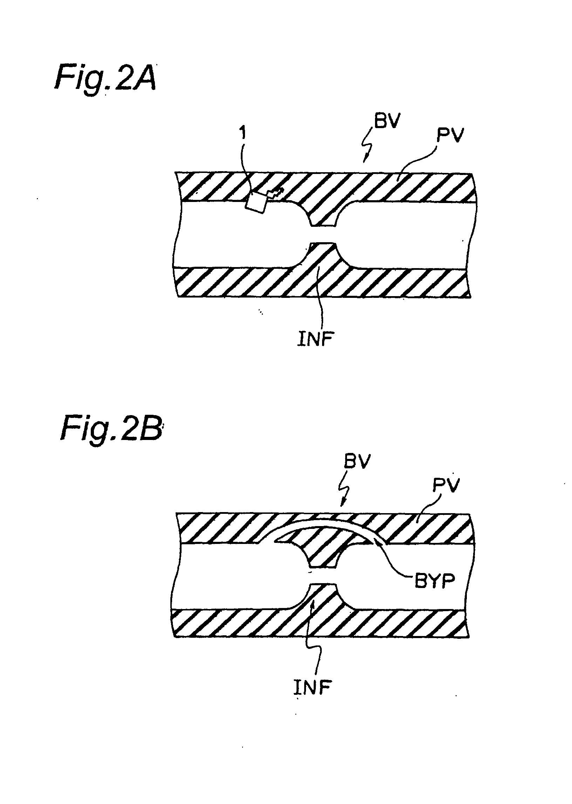

Referring Figs. 1A-1C and 2A-2B, a drug delivery system of the first embodiment according to the present invention will be described herein. The drug delivery system 1 includes, in general, a tank member (container) 2 and an anchor member (fixer) 3 extending from the tank member 2. Although not limited thereto, the tank member 2 has an outline of a substantially rectangular solid, in which a chamber 4 capable of holding a medicament such as the regenerative cells and/or factors and the anti-cancer drug is defined. The anchor member 3 is tapered along a longitudinal direction as indicated by "A" in Figs. 1A and 1B. Also, it is secured at one end to the tank member 2, and has a substantially sharp tip 6 at the other end. Also, the anchor member 3 has a plurality (e.g., four) of protruding portions 7 as shown in Figs. 1A-1C. Each of the protruding portions 7 has an outline of a part of a quadrangular pyramid, thus the anchor member 3 has a configuration combining a plurality of protruding portions 7, each of which outline is a partial quadrangular pyramid having sides different from one another. In addition, the anchor member 3 includes a channel 5 defined therein, in fluid communication with the chamber 4.

Both of the tank member 2 and the anchor member 3 are formed of material such as poly-lactic acid. Poly-lactic acid is composed of biocompatible and biodegradable polymer molecules, and hydrolyzed to be lactic acid, which is harmless to and metabolizable for a living body. Any other biodegradable material besides poly-lactic acid (including for example, glue, starch, protein, and glucose) may be used to form the tank member 2 and the anchor member 3. Thus, since the drug delivery system 1 according to the present invention is made of biodegradable material such as poly-lactic acid, advantageously it can be left or embedded within a body.

In soil, poly-lactic acid is degraded by aerobic bacteria to carbon dioxide and water, which can be photosynthesized to obtain poly-lactic acid. This constitutes a part of circulation circle named as a TCA circuit. Therefore, products made of poly-lactic acid can be disposal into the soil and realize a recycling friendly to the ecology, thereby showing good ecology affinity and recycling efficiency.

So far, the silicon-based material has often been used to produce a micro-machine that can be placed within a body, and in addition, the flexible polymer material such as polyimide and parylene is also utilized to produce such a micro-machine. However, the silicon-based material (e.g., Si, SiO₂, SiN), though chemically inactive to living tissue, cannot be evacuated by itself out of a body nor remained beside the blood vessel, because it may serve as a core causing a thrombus. The thrombus may block the blood vessel, as growing, and eventually lead a fatal disease such as a brain infarction. Similarly, since polyimide and parylene are not biodegradable material and not evacuated by itself out of a body, those cannot be placed within a body, neither. Therefore, like a drug delivery system according to the present invention, it is quite beneficial to choose biocompatible and biodegradable material such as poly-lactic acid as a stating material for producing a micro-machine product intended to be placed within a body. Poly-lactic acid has mechanical characteristics including the Young's modulus (rigidity) close to ones of polyimide and parylene, as illustrated in Table 1. Therefore, poly-lactic acid having biocompatibility/biodegradability and sufficient strength is referred to as "clean plastic".

**(Table 1)**

| Comparison of Characteristics Among Polyimide, Parylene, and Poly-lactic Acid | | | |
|---|---|---|---|
| | Polyimide | Parylene | Poly-lactic Acid |
| Young's Modulus [GPa] | 3 | 3.2 | 3.4 |
| Tensile Strength [MPa] | 120 | 70 | 64 |
| Tensile Breaking Elongation [%] | 10 | 200 | 4.1 |
| Grass Transition Point [C] | 310 | - | 61 |
| Melting Point [C] | 450 | 290 | 173 |
| Supplier | Dupont Microsystems | Union Carbide Corp. | Shimazu Corp. |
| Product No. | PIX-3476-4L | - | Lacty 5000 |
| Production Process | Spin Coating | CVD | Injection Molding |

The drug delivery system 1 according to the present invention has a sharp tip 6 so that, as illustrated in Fig. 2A, it can readily be penetrated into the vessel wall **PV** adjacent the infarction **INF** of the coronary vessel **BV** by means of a pinching device secured onto the catheter (not shown). Also, since the drug delivery system 1 is made of biodegradable material such as poly-lactic acid, advantageously, it can be left within the tissue of the blood vessel wall **PV** without any adverse effects to the body. Further, since the drug delivery system 1 has the anchor member 3, it can be retained for a substantial time period even in the blood vessel wall **PV** having rapid flow of blood. Once the drug delivery system 1 is embedded within the tissue of the blood vessel wall **PV**, poly-lactic acid forming the tank member 2 and the anchor member 3 is gently hydrolyzed to dissolve, the medicament (the regenerative cells and/or factors for regenerating the blood vessel) reserved in the tank member 2 can be released in small doses for a predetermined dosing period, e.g., one or weeks. During such a dosing period, as shown in Fig. 2B, the bypassing blood vessel **BYP** complementing the infarct vessel is formed. At this end, poly-lactic acid forming the tank member 2 and the anchor member 3 is completely hydrolyzed to be lactic acid which is not accumulated within the body, thus, the drug delivery system 1 has no need to be taken out. Although not shown, the tip 6 of the anchor member 3 may be formed with a thinner layer of poly-lactic acid than the remaining regions that can be dissolved at an earlier stage. This forms an opening at the tip 6, thereby allowing the medicament stored in the chamber 4 to be gently released through the channel 5 and the tip 6.

As described above, according to the present invention, the drug delivery system 1 can be placed in the blood vessel wall **PV** having rapid flow of blood for a prolonged time so that the stored regenerative cells and/or factors are slowly released and supplied to the body portion requiring the medicament, thereby efficiently forming the bypassing blood vessel **BYP** without the invasive surgical operation. «Manufacturing Process of DDS of First Embodiment»

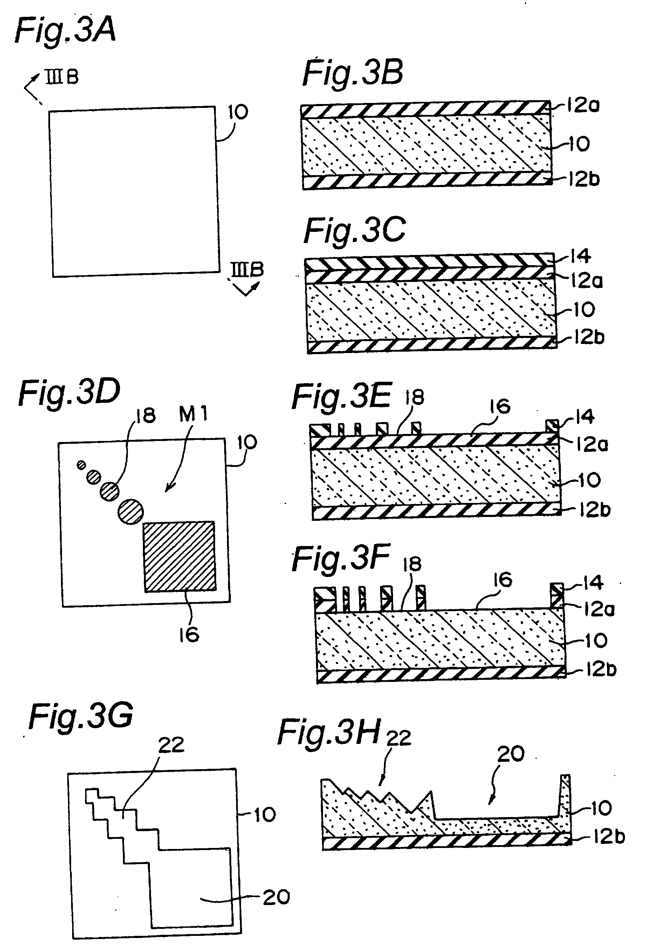

Next, referring to Figs. 3A-3H through 6A-6F, a manufacturing process of the drug delivery system of the first embodiment will be described herein.

Firstly, a pair of silicon substrates 10, 11 having principal surfaces of (100) crystal plane is prepared. As shown in Figs. 3A and 3B, one of the silicon substrates 10 is processed to have silicon dioxide (SiO₂) layers 12a, 12b on both surfaces and washed with sulfuric acid/hydrogen peroxide/water (H₂SO₄ : H₂O₂ = 3 : 1) and ammonium hydroxide/hydrogen peroxide/water (NH₄OH : H₂O₂ : H₂O = 1 : 1 : 5) for five minutes.

As shown in Fig. 3C, formed on the silicon dioxide layer 12a is a photoresist layer 14, which is baked at 90 degrees C for ten minutes.

As illustrated in Figs. 3D and 3E, the photoresist layer 14 is patterned with a mask M1. This mask M1 does not cover the regions of the photoresist layer 14 indicated by hatchings of Fig. 3D. Thus, the mask M1 uncovers a tank region 16 and a plurality of circle regions 18 discretely arranged in a line. Each of the circle regions 18 is designed so as to have smaller diameter as the center position thereof is away from the tank region 16.

In Fig. 3F, the silicon dioxide (SiO₂) layer 12a is reactive-ion etched with fluoroform gas (CHF₃) (etching condition: 5sccm, 5Pa, 100W, 1H).

Next, after the photoresist layer 14 is stripped off, the remaining silicon dioxide layer 12a is used as a mask for wet etching the silicon substrate 10 with potassium hydroxide (KOH) as an etchant (etching condition: 33weight%, 70 degrees C, 55 minutes). In general, silicon having a surface-orientation dependency (etch anisotropy) with the etchant of potassium hydroxide is etched along the orientation perpendicular the (111) crystal plane of silicon. To this result, as shown in Figs. 3G and 3H, a plurality of recesses, each of which has an outline of a flipped quadrangular pyramid, are overlapped one another so that an anchor recess 22 is formed. Similarly, formed in the tank region 16 is a tank recess 20, which is in fluid communication with the anchor recess 22.

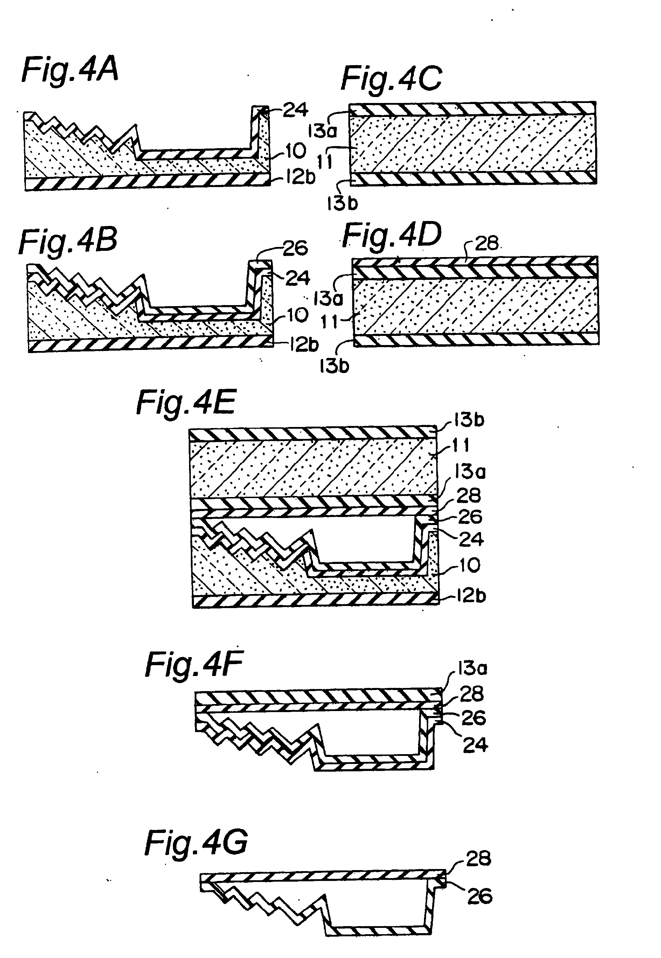

After again forming a silicon dioxide (SiO₂) layer 24 on the silicon substrate 10 having the tank recess 20 and the anchor recess 22 (referred to as "the first silicon substrate 10", herein) as illustrated in Fig. 4A, a thin layer 26 of poly-lactic acid is formed thereon, as shown in Fig. 4B.

Examples to form the thin layer 26 of poly-lactic acid include a solvent-dissolution spin coating and a heat-melt spin coating. In the solvent-dissolution spin coating, a solution obtained by thoroughly dissolving solid phase of poly-lactic acid with solvent such as chloroform (CHCl₃) is applied on the silicon substrate and spin-coated. Also, the solvent is fully evaporated so as to form the thin layer solely made of poly-lactic acid. These steps may be repeated to control the thickness of the thin layer of poly-lactic acid as desired. On the other hand, in the heat-melt spin coating, liquid phase of poly-lactic acid obtained by heating to melt solid phase of poly-lactic acid is applied on the silicon substrate and spin-coated. Then, it is cooled down by leaving at room temperature so that the thin layer of poly-lactic acid is formed. Any other processes well known by those skilled in the art may be used to form the thin layer of poly-lactic acid.

Similarly, formed on both surfaces of another intact silicon substrate rather than the first silicon substrate 10, which is referred to as the second silicon substrate 11, are silicon dioxide (SiO₂) layers 13a, 13b, as shown in Fig. 4C.

In Fig. 4D, a thin layer 28 of poly-lactic acid is also formed on one surface of the second silicon substrate 11.

Next, as illustrated in Fig. 4E, the first and second silicon substrates 10, 11 are laminated so that the thin layers 26, 28 of poly-lactic acid face to each other. Then, the first and second silicon substrates 10, 11 are securely bonded to each other by heating thereof close to the melting point of poly-lactic acid. Thus, the space is defined between the thin layers 26, 28 of poly-lactic acid to form the chamber 4 of the tank member 2 and the channel 5 of the anchor member 3.

Next, the silicon dioxide (SiO₂) layers 12b, 13b are reactive-ion etched with fluoroform gas (CHF₃) (etching condition: 5sccm, 5Pa, 100W, 1H). Then, the silicon substrate 10, 11 are removed, remaining the silicon dioxide (SiO₂) layers, for example, by wet etching with tetra-methyl ammonium hydroxide (TMAH) or by ion-reactive etching with sulfur hexafluoride (SF₆).

Lastly, as shown in Fig. 4G, the silicon dioxide (SiO₂) layers 13a, 24 are stripped off, for example, by wet etching with hydrofluoric acid (HF) or by ion-reactive etching with fluoroform gas (CHF₃) so as to obtain the drug delivery system 1 of the first embodiment.

The medicament is injected into the chamber 4 of the drug delivery system by use of any appropriate ways. For example, a through-hole is made at a suitable position of the tank member 2 or the anchor member 3 extending through the chamber 4 or the channel 5 with a focused-ion-beam system (FIB), through which the chamber 4 is filled up with the medicament. Then, the thin layer of poly-lactic acid around the through-hole is heated and melted to occlude the through-hole.

As described above, the drug delivery system 1 according to the present invention can be manufactured based upon a micro-machine technology applying the fine processing technology of a semiconductor integrated circuit device. Based upon the fine processing technology currently available, the processing accuracy in the order of nanometer for a submicron structure can be realized. Therefore, according to the micro-machine technology, the drug delivery system 1 having any desired dimension and configuration can be manufactured in a precise manner and at a reasonable cost.

### «Modification 1: Alternative Manufacturing Process»

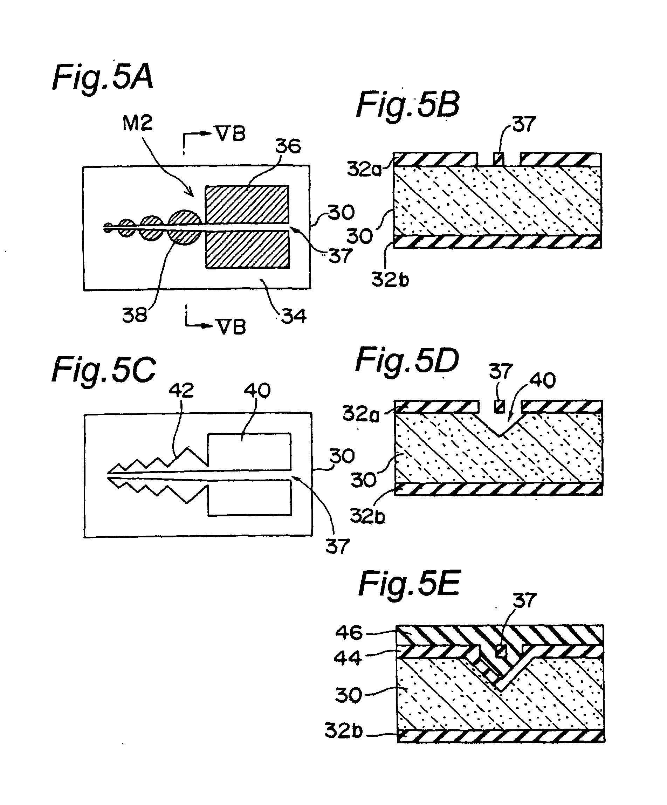

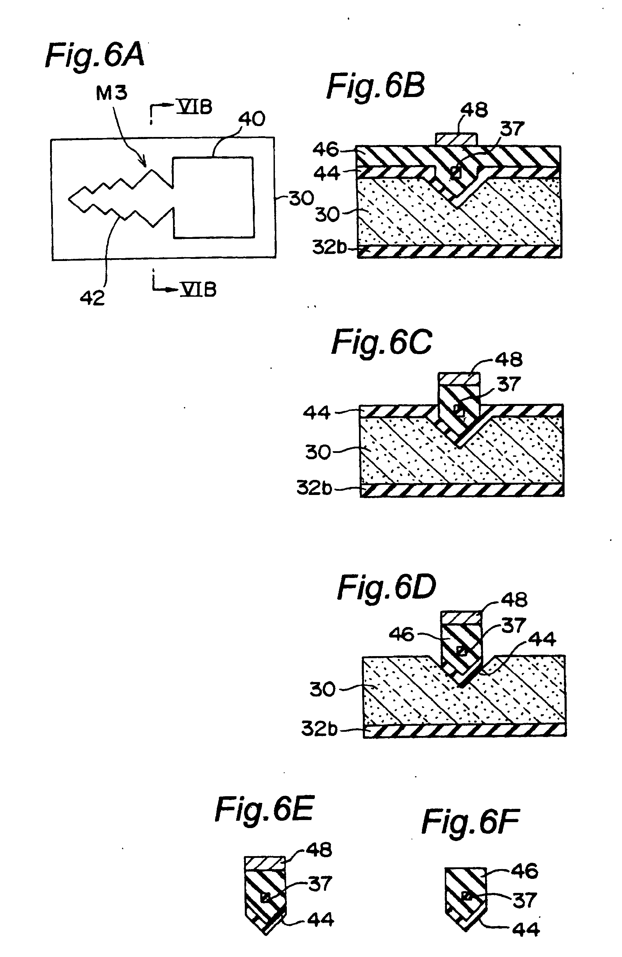

Referring to Figs. 5A-5E and 6A-6F, an alternative manufacturing process of the drug delivery system of the first embodiment (first modification) will be described herein.

In the alternative manufacturing process, firstly, a silicon substrate 30 having principal surface of (100) crystal plane is prepared. Although not shown in the drawing as being similar to the above-mentioned process, a silicon substrates 30 is processed to have silicon dioxide (SiO₂) layers 32a, 32b on both surfaces and washed with sulfuric acid/hydrogen peroxide/water (H₂SO₄ : H₂O₂ = 3 : 1) and ammonium hydroxide/hydrogen peroxide/water (NH₄OH : H₂O₂ : H₂O = 1 : 1 : 5) for five minutes. Then, a photoresist layer 34 is formed on the silicon dioxide layer, which is baked at 90 degrees C for ten minutes.

Next, a mask M2 shown in Fig. 5A is used to pattern the photoresist layer 34. This mask M2 does not cover the regions of the photoresist layer 34 indicated by hatchings of Fig. 5A. Thus, the mask M2 uncovers a tank region 36 and a plurality of circle regions 38 discretely arranged in a line, except of a bridge region 37 extending through the tank region 36 and the circle regions 38. Also, each of the circle regions 38 is formed so as to have smaller diameter as the center position thereof is away from the tank region 36.

In Fig. 5B, the silicon dioxide (SiO₂) layer 32a is reactive-ion etched with fluoroform gas (CHF₃) (etching condition: 5sccm, 5Pa, 100W, 1H), and the photoresist layer 14 is stripped off. Then, the remaining silicon dioxide layer 32a is used as a mask for wet etching the silicon (Si) substrate 30 with potassium hydroxide (KOH) as an etchant (etching condition: 33 weight%, 70 degrees C, 55 minutes). As described above, silicon has a surface-orientation dependency (etch anisotropy) with the etchant of potassium hydroxide. Therefore, as illustrated in Figs. 5C and 5D, a plurality of recesses, each of which has an outline of a flipped quadrangular pyramid, are overlapped one another so that an anchor recess 42 is formed. Similarly, formed in the tank region 36 is a tank recess 40, which is in fluid communication with the anchor recess 42. It should be noted that the silicon dioxide (SiO₂) layer is still remained in the bridge region 37, which eventually forms the chamber 4 and the channel 5.

In Fig. 5E, another silicon dioxide (SiO₂) layer 44 is formed on the silicon substrate 30 having the tank recess 40 and the anchor recess 42, and then another layer 46 of poly-lactic acid is formed thereon by pouring heated and melted poly-lactic acid onto the silicon substrate 30 (including the tank recess 40 and the anchor recess 42).

Next, although not shown, an evaporated aluminum (Al) layer is formed on the poly-lactic acid layer 46, on which another photoresist layer is formed. A mask M3 shown in Fig. 6A is used to pattern the photoresist layer. The mask M3 is formed over the tank recess 40 including the bridge region 37 and the anchor recess 42. The aluminum layer is etched by phosphoric acid (H₃PO₄) or mixed acid with the mask M3 to obtain a patterned aluminum thin layer 48 shown in Fig. 6B.

As illustrated in Fig. 6C, after removing the photoresist layer, the patterned aluminum thin layer 48 is used as a mask to remove (ash) the poly-lactic acid layer by plasma-etching with oxygen gas (O₂), leaving the aluminum thin layer 48. Also, the silicon dioxide (SiO₂) layers 44 is reactive-ion etched with fluoroform gas (CHF₃) (etching condition: 5sccm, 5Pa, 100W, 1H).

Next, in Fig. 6D, an etchant reactive with silicon (Si) but inactive with silicon dioxide (SiO₂) is used to etch the silicon substrate 30. For example, the silicon substrate 30 is wet etched with tetra-methyl ammonium hydroxide (TMAH) or ion-reactive etched with sulfur hexafluoride (SF₆).

In Fig. 6E, an etchant active with aluminum but inactive with poly-lactic acid and silicon dioxide (SiO₂) such as phosphoric acid (H₃PO₄) and mixed acid is used to etch the aluminum thin layer 48.

Lastly, the drug delivery device 1 is bathed into an etchant active with silicon dioxide (SiO₂) but inactive with poly-lactic acid such as hydrofluoric acid (HF), so that the silicon dioxide (SiO₂) layer 44 beneath poly-lactic acid and silicon dioxide (SiO₂) within the bridge region 37 are completely removed. Thus, the drug delivery system 1 is obtained solely made of poly-lactic acid.

The drug delivery device 1 has openings (not shown) at positions corresponding to both ends of the bridge region 37 shown in Fig. 5C. After filling up with the medicament through the openings, the thin layer of poly-lactic acid forming the tank member 2 and the anchor member 3 is heated and melted to occlude the openings.

### «Drug Delivery System of Second Embodiment»

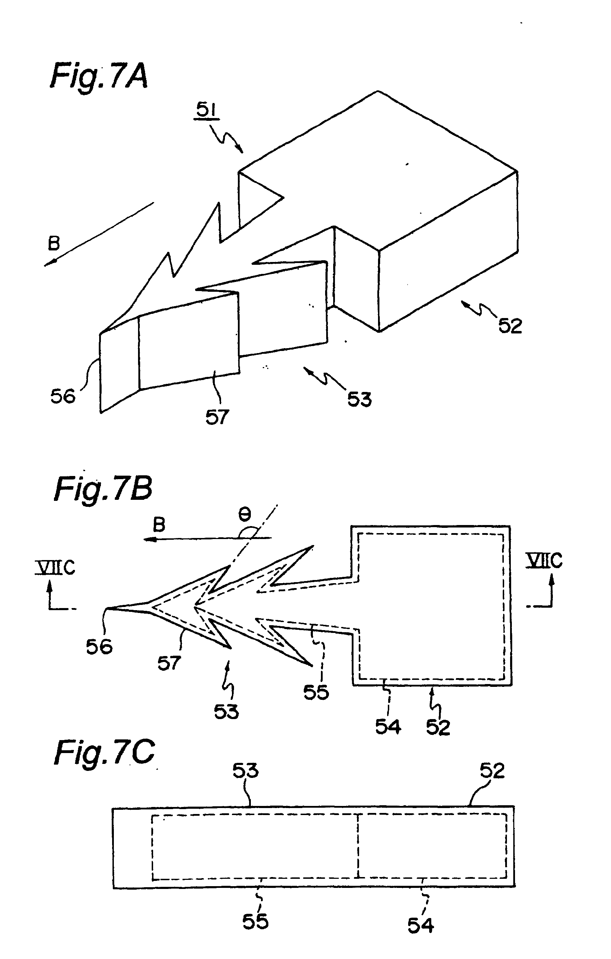

Referring to Figs. 7A-7C, a drug delivery system of the second embodiment according to the present invention will be described herein. Similar to the first embodiment, the drug delivery system 51 of the present embodiment includes, in general, a tank member (container) 52 and an anchor member (fixer) 53 extending from the tank member 52. Although not limited thereto, the tank member 52 has an outline of a rectangular solid, in which a chamber 54 capable of holding a medicament such as the regenerative cells and/or factors and the anti-cancer drug is defined. The anchor member 53 is tapered along a longitudinal direction as indicated by "B" in Figs. 7A and 7B. Also, it is secured at one end to the tank member 52, and has a substantially sharp tip 56 at the other end. Also, the anchor member 53 has a plurality (e.g., four) of protruding portions 57 as shown in Figs. 7A and 7B. Each of the protruding portions 57 has an outline of a part of a triangular prism. Either one, or preferably both of the side surfaces of the triangular prisms extend in a direction inclined at an obtuse angle (θ) to the longitudinal direction of "B". In addition, the anchor member 53 includes a channel 55 defined therein, in fluid communication with the chamber 54.

Also, similar to the first embodiment, both of the tank member 52 and the anchor member 53 are formed of biodegradable material such as poly-lactic acid, and the sharp tip 56 is formed. Therefore, the drug delivery system 51 can be embedded in a desired portion of a living body where the treatment is required, providing no adverse effect to the body. Also, the protruding portions 57 of the anchor member 53 extend in a direction inclined at an obtuse angle (θ) to the embedded direction of "B" indicated in Figs. 7A and 7B, so that once embedded into the treatment portion, they engage with the peripheral tissue thereof. This prevents the drug delivery system 51 from being released from the treatment portion and allows it to be secured thereon for a long time even where the treatment portion has a rapid flow of body fluid such as blood. Thus, poly-lactic acid forming the tank member 52 and the anchor member 53 of the drug delivery system 51 is gently hydrolyzed to dissolve, the medicament reserved in the tank member 52 can be released in small doses for a predetermined dosing period. Although not shown, the side surfaces of the protruding portions 57 of the anchor member 53 may be formed with a thinner layer of poly-lactic acid than the remaining regions so as to be dissolved at an earlier stage. This forms openings at the protruding portions 57, thereby allowing the medicament stored in the chamber 54 to be released through the channel 55 and the side surfaces of the protruding portions 57. Therefore, like the first embodiment, the drug delivery system 51 is used to form the bypassing blood vessel **BYP** complementing the infarct vessel as shown in Fig. 2B.

### «Manufacturing Process of DDS of Second Embodiment»

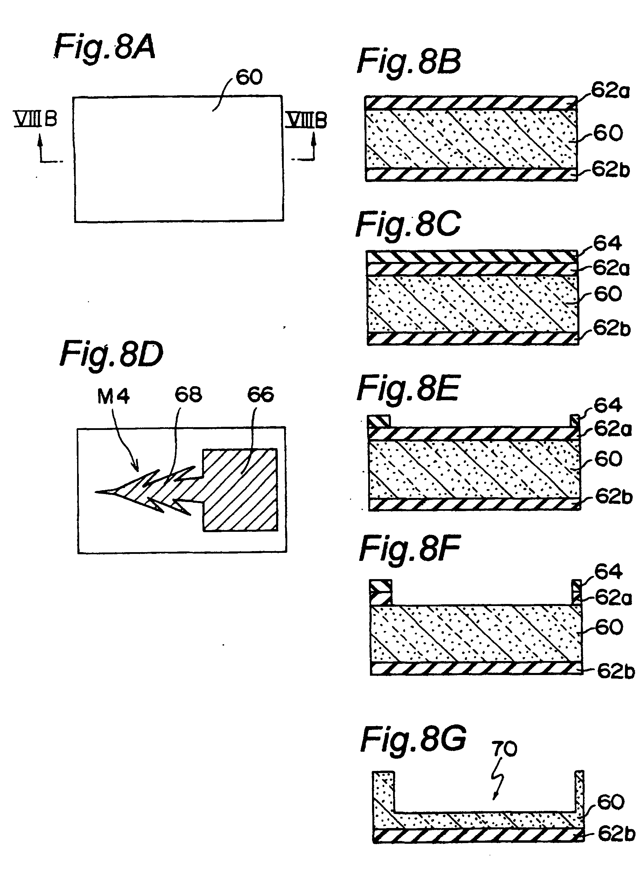

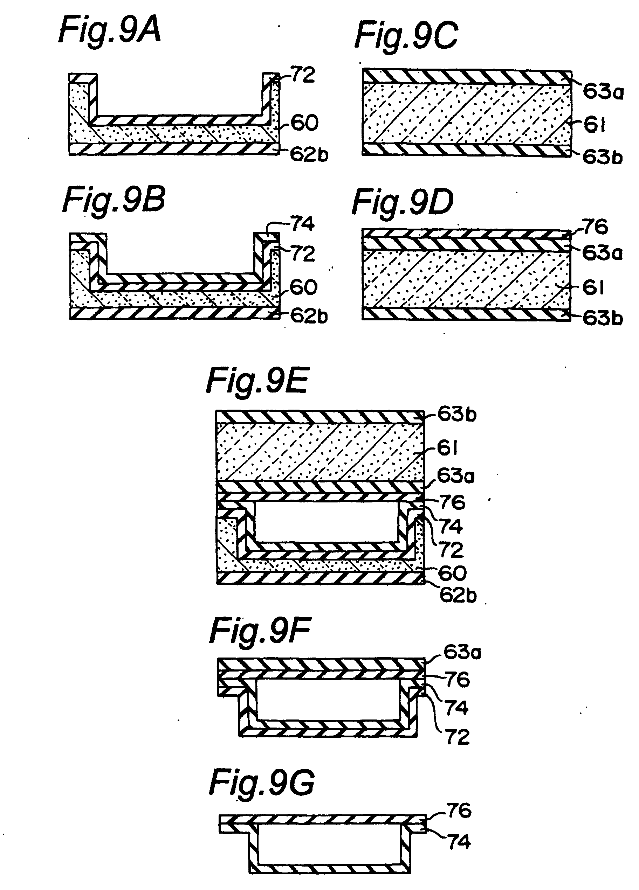

Next, referring to Figs. 8A-8G and 9A-9G, a manufacturing process of the drug delivery system of the second embodiment will be described herein.

Firstly, a pair of silicon substrates 60, 61 is prepared. As shown in Figs. 8A and 8B, one of the silicon substrates 60 is processed to have silicon dioxide (SiO₂) layers 62a, 62b on both surfaces and washed with sulfuric acid/hydrogen peroxide/water (H₂SO₄ : H₂O₂ = 3 : 1) and ammonium hydroxide/hydrogen peroxide/water (NH₄OH : H₂O₂ : H₂O = 1 : 1 : 5) for five minutes.

As shown in Fig. 8C, applied on the silicon dioxide layer 62a is a photoresist layer 64, which is baked at 90 degrees C for ten minutes.

As illustrated in Figs. 8D and 8E, the photoresist layer 64 is patterned with a mask M4. This mask M4 does not cover the regions of the photoresist layer 64 indicated by hatchings of Fig. 8D. Thus, the mask M4 uncovers a tank region 66 having a substantially rectangular shape and an anchor region 68 having a shape overlapping two pairs of flukes.

In Fig. 8F, the silicon dioxide (SiO₂) layer 62a is reactive-ion etched with fluoroform gas (CHF₃) (etching condition: 5sccm, 5Pa, 100W, 1H).

Next, after the photoresist layer 64 is stripped off, the remaining silicon dioxide layer 62a is used as a mask for reactive-ion etching the silicon substrate 60 with sulfur hexafluoride (SF₆) (etching condition: 50sccm, 20Pa, 100W, 45minutes). To this result, as shown in Figs. 8G, a recess 70 of a predetermined depth having a tank recess and the an anchor recess is formed in the tank region 66 and the anchor region 68 in fluid communication with each other.

After again forming a silicon dioxide (SiO₂) layer 72 on the silicon substrate 60 having the recess 70 (referred to as "the first silicon substrate 60", herein) as illustrated in Fig. 9A, a thin layer 74 of poly-lactic acid is formed thereon as shown in Fig. 9B, by the above-mentioned solvent-dissolution spin coating or the heat-melt spin coating.

Also, as shown in Fig. 9C, silicon dioxide (SiO₂) layers 63a, 63b are formed on both surfaces of another intact silicon substrate 61 rather than the first silicon substrate 60, which is referred to as the second silicon substrate.

In Fig. 9D, a thin layer 76 of poly-lactic acid is also formed on one surface of the second silicon substrate 61.

Next, as illustrated in Fig. 9E, the first and second silicon substrates 60, 61 are laminated so that the thin layers 74, 76 of poly-lactic acid are faced to each other. Then, the first and second silicon substrates 60, 61 are securely bonded to each other by heating thereof close to the melting point of poly-lactic acid.

Next, in Fig. 9F, the silicon dioxide (SiO₂) layers 62b, 63b are reactive-ion etched with fluoroform gas (CHF₃) (etching condition: 5sccm, 5Pa, 100W, 1H). Then, the silicon substrate 60, 61 are removed, remaining the silicon dioxide (SiO₂) layers 63a, 72, for example, by wet etching with tetra-methyl ammonium hydroxide (TMAH) or by ion-reactive-etching with sulfur hexafluoride (SF₆).

Lastly, as shown in Fig. 9G, the silicon dioxide (SiO₂) layers 63a, 72 are stripped off with an etchant inreactive with poly-lactic acid but reactive with silicon dioxide (SiO₂). For example, it is wet etched with hydrofluoric acid (HF) or by dry-etched with fluoroform gas (CHF₃) so as to realize the drug delivery system 51 of the second embodiment.

Also, the medicament is injected into the drug delivery system chamber by use of any appropriate means. For example, a through-hole is made at a suitable position of the tank member 52 or the anchor member 53 extending through the chamber 54 or the channel 55 with a focused-ion-beam system (FIB), through which the chamber 54 is filled up with the medicament. Then, the thin layer of poly-lactic acid around the through-hole is heated and melted to occlude the through-hole.

### «Modification 2: Alternative Manufacturing Process»

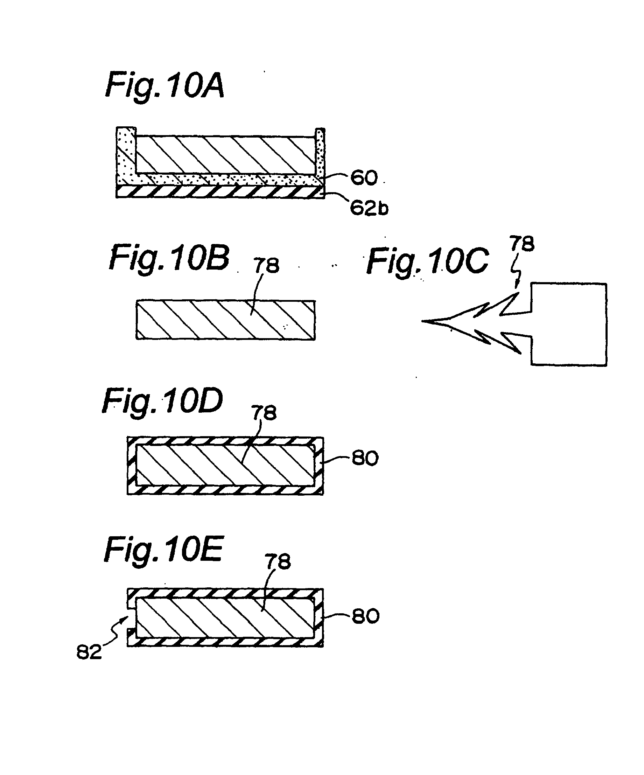

With reference of Figs. 10A-10E, an alternative manufacturing process of the drug delivery system of the second embodiment (second modification) will be described herein.

In the alternative manufacturing process, firstly, a silicon substrate 60 is prepared and processed as described above with reference of Figs. 8A-8G.

Then, the recess 70 having a predetermined depth in the tank region 66 and the anchor region 68 of the mask M4 is filled up with melted aluminum.

Silicon is etched off to obtain a micro molding die 78 of aluminum (Al) having a configuration similar to the drug delivery system of the second embodiment, as illustrated in Figs. 10B and 10C.

The micro molding die 78 is immersed into melted poly-lactic acid and drawn up, and then left at room temperature to form a thin layer 80 of poly-lactic acid encompassing the micro molding die 78.

Similarly, a through-hole 82 is made at a suitable position of the tank member 52 or the anchor member 53 with a focused-ion-beam system (FIB) so that a portion of the micro molding die 78 is exposed as shown in Fig. 10E. Then, the micro molding die 78 is immersed into an etchant solution active with aluminum but inactive with poly-lactic acid such as phosphoric acid (H₃PO₄) to form the drug delivery system 51 having an opening 82. Lastly, the medicament is injected into the drug delivery system through the opening 82, and then, the thin layer of poly-lactic acid around the opening 82 is occluded by heating and melting.

### «Drug Delivery System of Third Embodiment»

A drug delivery system of the third embodiment according to the present invention will be described herein. This drug delivery system 51 having a structure similar to one of the second embodiment except that the anchor member 53 has no channel 55 in fluid communication with the chamber 54 of the tank member 52, thus duplicate description will be eliminated herein.

According to the drug delivery system 51 so structured, the chamber 54 of the tank member 52 can preserve a medicament such as the regenerative cells and/or factors and the anti-cancer drug. Also, once embedded into the treatment portion, the protruding portions 57 of the anchor member 53 engage with the peripheral tissue thereof. This prevents the drug delivery system 51 from being released from the treatment portion and allows it to be secured thereon for a long time even where the treatment portion has a rapid flow of body fluid such as blood. Thus, as poly-lactic acid forming the tank member 52 and the anchor member 53 of the drug delivery system 51 is gently hydrolyzed to dissolve, the medicament reserved in the tank member 52 can be released in small doses for a predetermined dosing period.

### «Manufacturing Process of DDS of Third Embodiment»

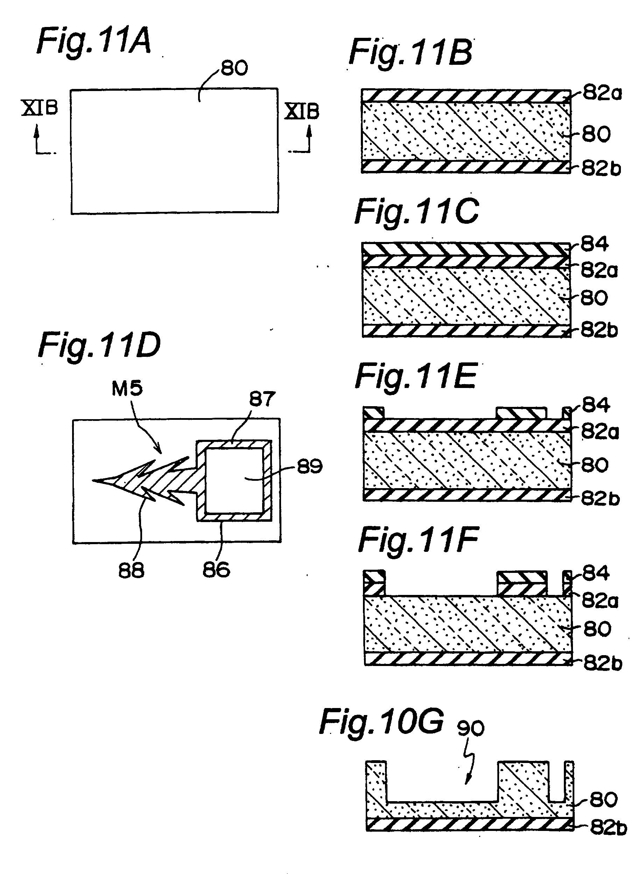

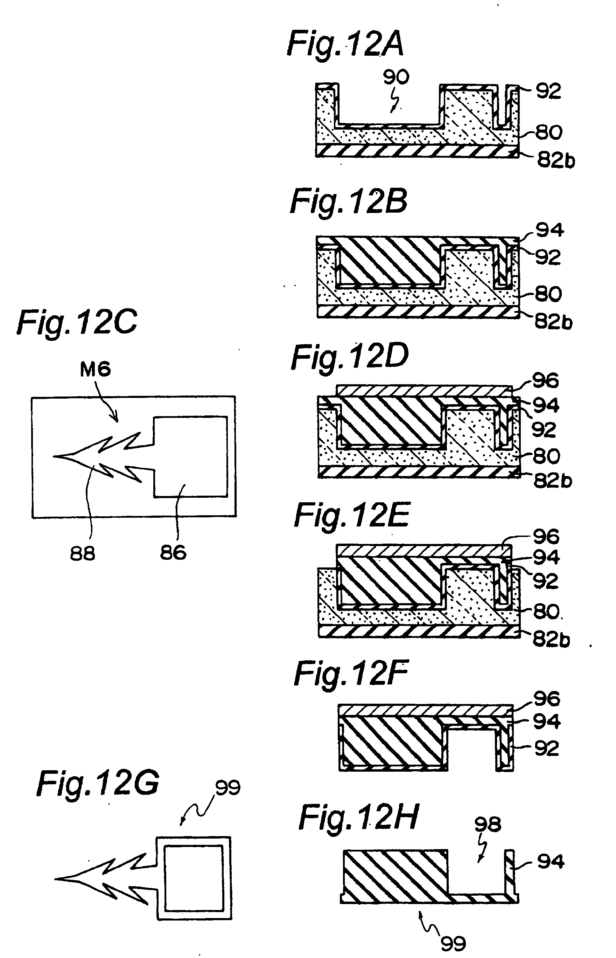

Referring to Figs. 11A-11G and 12A-12H, a manufacturing process of the drug delivery system 51 of the third embodiment will be described herein.

In the manufacturing process, a silicon substrate 80 is prepared and processed to have silicon dioxide (SiO₂) layers 82a, 82b on both surfaces and washed with sulfuric acid/hydrogen peroxide/water (H₂SO₄ : H₂O₂ = 3 : 1) and ammonium hydroxide/hydrogen peroxide/water (NH₄OH : H₂O₂ : H₂O = 1 : 1 : 5) for five minutes..

Then, a photoresist layer 84 is applied on the silicon dioxide layer, which is baked at 90 degrees C for ten minutes.

Next, a mask M5 shown in Fig. 11D is used to pattern the photoresist layer 84. This mask M5 does not cover the regions of the photoresist layer 34 indicated by hatchings of Fig. 11D. Thus, the mask M5 uncovers a peripheral portion 87 of a tank region 86 and an anchor region 88 having a shape overlapping two pairs of flukes. However, it covers a middle portion 89 of the tank region 86.

In Fig. 11F, the silicon dioxide (SiO₂) layer 82a is reactive-ion etched with fluoroform gas (CHF₃) (etching condition: 5sccm, 5Pa, 100W, 1H).

After the photoresist layer 84 is stripped off, the remaining silicon dioxide layer 32a is used as a mask for reactive-ion etching the silicon (Si) substrate with sulfur hexafluoride (SF₆) (etching condition: 50sccm, 20Pa, 100W, 45minutes). To this result, as shown in Figs. 11G, the silicon substrate is recessed in the peripheral portion 87 of the tank region 86 and the anchor region 88 to have a recess 90 of a predetermined depth in fluid communication to each other.

As shown in Fig. 12A, a silicon dioxide (SiO₂) layer 92 is again formed on the silicon substrate 80. Then, as shown in Fig. 12B, the recess 90 formed in the peripheral portion 87 of the tank region 86 and the anchor region 88 is filled up with melted poly-lactic acid, so as to form a thin layer 94 of poly-lactic acid.

Next, although not shown in the drawing, aluminum (Al) is deposited on the thin layer 94 of poly-lactic acid to form an aluminum layer, on which in turn a photoresist layer is formed. Then, a mask shown in Fig. 12C is used to cover the tank region 86 and the anchor region 88. The aluminum layer is etched by phosphoric acid (H₃PO₄) or mixed acid with the mask M6 to obtain a patterned aluminum thin layer 48 shown in Fig. 12D.

As illustrated in Fig. 12E, the patterned aluminum thin layer 96 is used as a mask to remove (ash) the poly-lactic acid layer by plasma-etching with oxygen gas (O₂). Also, the silicon dioxide (SiO₂) layer 94 is reactive-ion etched with fluoroform gas (CHF₃) (etching condition: 5sccm, 5Pa, 100W, 1H).

Next, in Fig. 12F, an etchant active with silicon (Si) but inactive with silicon dioxide (SiO₂) is used to etch the silicon substrate 80. For example, the silicon substrate 30 is wet etched with tetra-methyl ammonium hydroxide (TMAH) or ion-reactive etched with sulfur hexafluoride (SF₆).

In Figs. 12G and 12H, an etchant reactive with aluminum but inactive with poly-lactic acid and silicon dioxide (SiO₂), such as phosphoric acid (H₃PO₄) and mixed acid is used to etch the aluminum thin layer 96. Lastly, the silicon dioxide layer 82b beneath poly-lactic acid is etched and removed by the reactive-ion etching with fluoroform gas (CHF₃) (etching condition: 5sccm, 5Pa, 100W, 1H) so as to obtain an intermediate structure solely made of poly-lactic acid shown in Fig. 12H. The structure of Fig. 12H is illustrated as being flipped of Fig. 12F.

The structure solely made of poly-lactic acid has an opening 98 uncovered in the region corresponding to the tank member 52, allowing the medicament to be injected through the opening 98. After injection, another thin layer of poly-lactic acid (not shown) is used to cover the opening 98, and then those are sealed by heating and depressing to each other so as to obtain the drug delivery system 51 having the medicament sealed within the chamber 54.

The anchor member 53 of the third embodiment manufactured by the present process has no channel 55 and is filled with poly-lactic acid, therefore, it is manufactured as being a solid type of the drug delivery system. However, a hollow type of the drug delivery system, similar to one of the second embodiment, can also be produced by designing the mask M5 to cover a middle portion of the anchor region 88 as well.

In addition, the tank member 52 may be formed to have no chamber and fully filled up with poly-lactic acid as being solid type of the tank member as well as the anchor member 53. However, in this case, the medicament should have been impregnated within the poly-lactic acid material composing the drug delivery system in advance. Once the drug delivery system 51 made of such a poly-lactic acid material containing the medicament is placed within a body, the medicament impregnated therein is slowly released as the poly-lactic acid material is gently hydrolyzed to dissolve, thus the same advantage can be expected as the above embodiments.

### «Drug Delivery System of Fourth Embodiment»

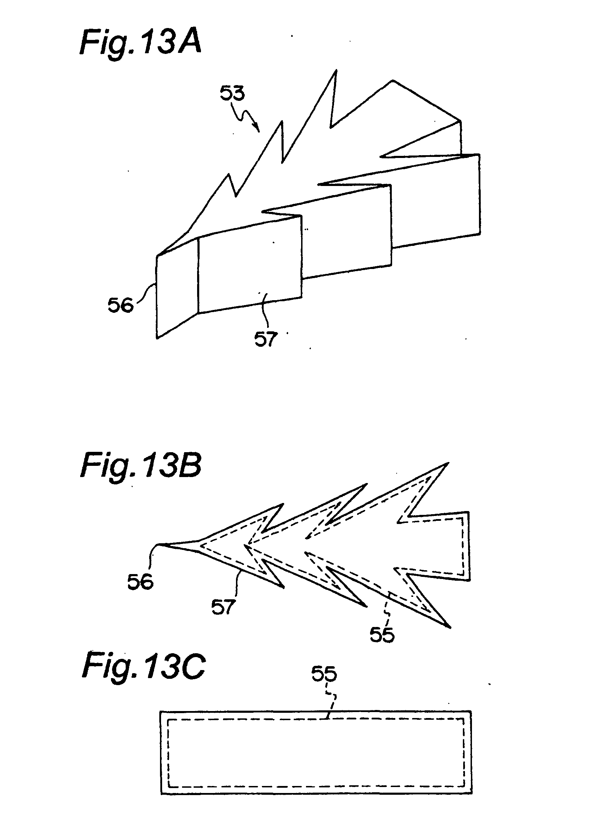

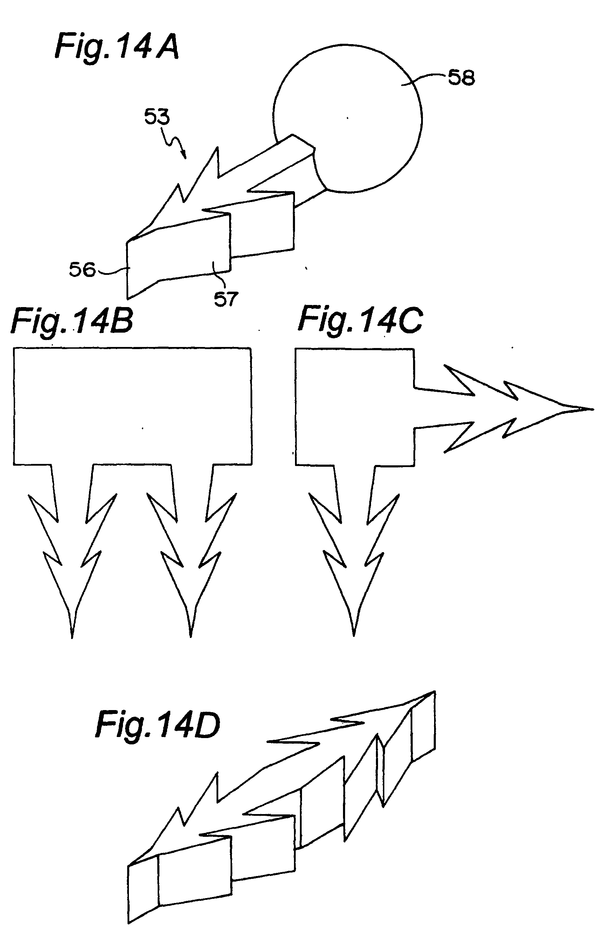

Referring to Figs. 13A-13C and 14A-14D, a drug delivery system of the fourth embodiment according to the present invention will be described herein. This drug delivery system 51 having a structure similar to one of the second embodiment except that the tank member 52 is eliminated, thus duplicate description will be eliminated herein.

In the drug delivery system 51, the chamber 54 holding the medicament such as the regenerative cells and/or factors and the anti-cancer drug is defined within the anchor member 53. Although Figs. 13A-13C illustrate the drug delivery system 51 having six protruding portions 57, it may include more or less of protruding portions 57. As those skilled in the art would realize, the drug delivery system 51 may be formed by any manufacturing processes described above in the second embodiment.

Also, although the drug delivery system 51 of the present embodiment is produced as a hollow type of the anchor member 53 as the second embodiment, it may also be designed as a sold type of the anchor member 53 as the third embodiment. In this case, the solid type of the anchor member 53 is formed of poly-lactic acid material containing the medicament as described above in the third embodiment.

The drug delivery system 51 solely made from the hollow and solid type of the anchor member 53 can be modified in many applications. For example, a solid medicine 58 in a tablet form may directly be fixed on one end opposite to the tip 56.

Alternatively, the tank member having the chamber made of poly-lactic acid may separately be formed by any processes as those skilled in the art can realize, and then pressed and adhered onto the anchor member 53 of the present embodiment to form the drug delivery system. Besides, the anchor member and the tank member of poly-lactic acid may readily be adhered with other appropriate biodegradable material such as glue, starch, protein, and glucose.

Also, a plurality of anchor members may be adhered on a single tank member extending in the same direction as shown in Fig. 14B or extending in the different directions as illustrated in Fig. 14C. Further, as shown in Fig. 14D, two of the anchor members are combined so that the sharp tips are arranged on both ends in the longitudinal direction.

### «Drug Delivery System of Fifth Embodiment»

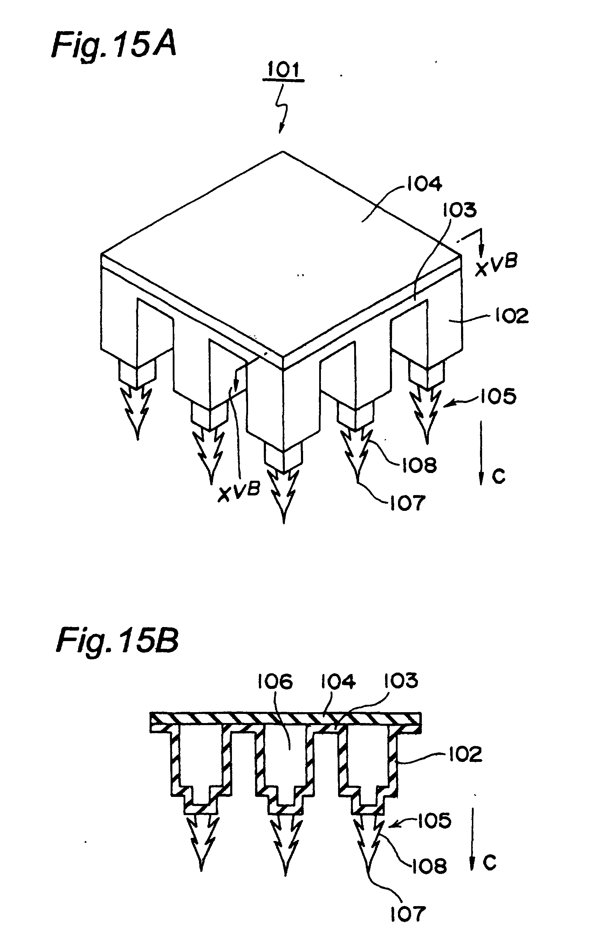

Referring to Figs. 15A-15B, a drug delivery system according to the fifth embodiment will be described herein. In Figs. 15A-15B, the drug delivery system 101 includes, in general, a plurality of tank members (containers) 102 (five of nine tank members are shown herein), a connecting member (connector) 103 for connecting adjacent tank members 102, a cap member 104 for hermetically sealing each of the tank members 102, and a plurality of anchor members (fixers) 105 extending from the respective one of the tank members 102. Each of the tank members 102 has an outline of a rectangular solid, in which a chamber 106 capable of holding a medicament is defined. Each of the anchor members 105 is tapered along a longitudinal direction as indicated by "C" in Figs. 15A-15B, and each has one end secured to the respective one of the tank members 102, and the other end having a sharp tip 107. Also, the anchor member 103 has a plurality (e.g., four) of protruding portions 108 as shown in Figs. 15A-15B. Each of the protruding portions 108 may have an outline similar to those of the second and third embodiments and may be designed as being solid or hollow.

All of the components of the drug delivery system 101 are made of biodegradable material such as poly-lactic acid similar to the first to fourth embodiments, and each of the anchor members 105 has the sharp tip 107. Therefore, the drug delivery system 101 can be embedded into the desired portion (treatment portion) without any adverse effects to a body. Also, since each of the anchor members 105 has the protruding portions 108, once embedded into the treatment portion, the protruding portions 108 engage with the peripheral tissue thereof. This prevents the drug delivery system 101 from being released from the treatment portion and allows it to be secured thereon for a long time even where the treatment portion has a rapid flow of body fluid such as blood. Thus, poly-lactic acid forming the tank member 102 and the anchor member 105 of the drug delivery system 101 is gently hydrolyzed to dissolve, the medicament reserved in the tank member 52 can be released in small doses for a predetermined dosing period.

Although not shown, the side surface of each of the tank members 102 may have a layer of poly-lactic acid adjusted such that the timing for releasing the medicaments stored within the tank members 102 is controlled. Thus, various types of medicaments in different tank members 102 can be released at the different timings. For example, the regenerating cells for inducing the regeneration of the blood vessel are stored in the chamber 106 of the tank member 102 having the thinner side surface, and the regenerating factors for growing the regenerating cells are held within the chamber 106 of the tank member 102 having thicker side surface, which releases the medicament at a later timing. Thus, the regenerating cells induces the regeneration of the blood vessel and after some appropriate time has passed, the regenerating factors control the regenerating cells to regenerate the blood vessel in an effective manner. Also, a plurality of the tank members 102 may be designed such that the same type the medicament is released at different timings. This allows a longer dosing period of the same medicament.

### «Manufacturing Process of DDS of Fifth Embodiment»

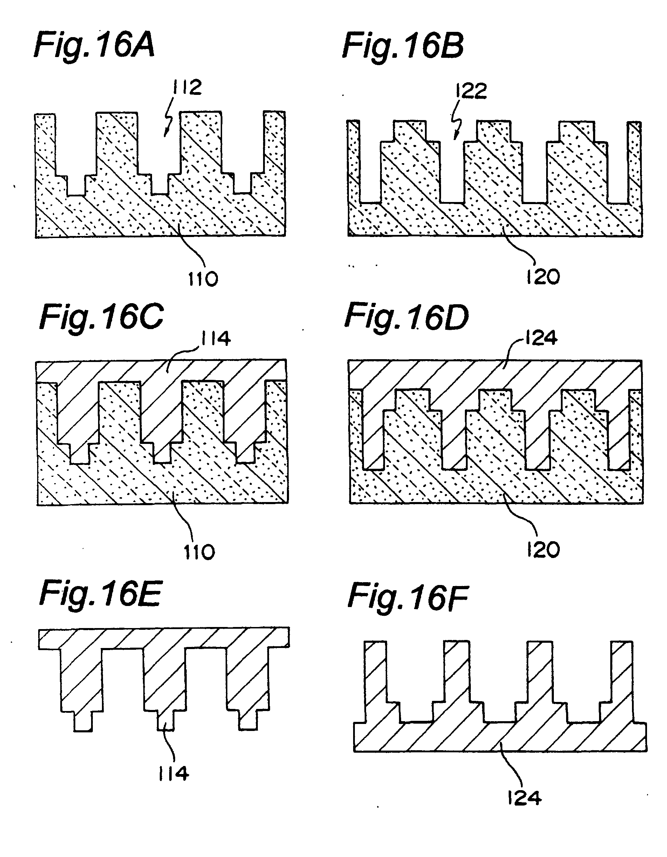

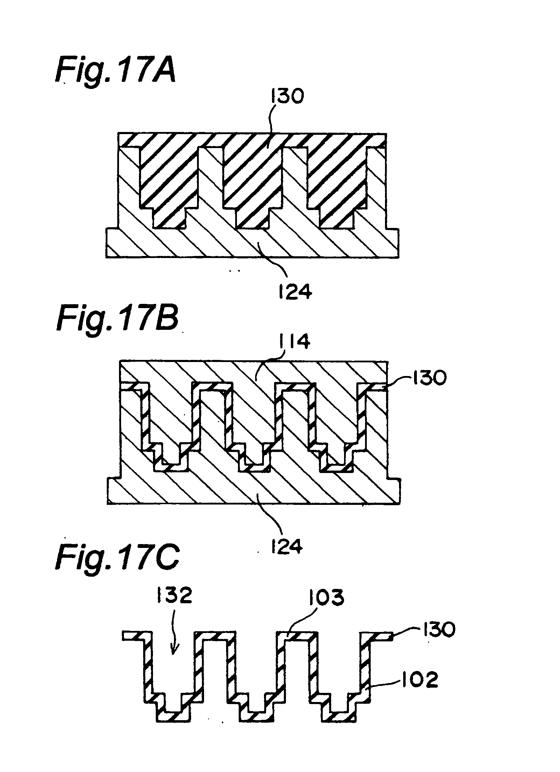

Next, referring to Figs. 16A-16F and 17A-17C, a manufacturing process of the drug delivery system of the fifth embodiment will be described herein.

Firstly, a pair of silicon substrates 110, 120 is prepared. The silicon substrates 110, 120 are processed with the micro photolithography as described above to form recesses 112, 122 of different shapes thereon as illustrated in Fig. 16A and 16B, respectively.

Next, melted aluminum is molded into the recesses 112, 122 as illustrated in Figs. 16C and 16D.

The silicon substrate 110, 120 are removed by wet etching with tetra-methyl ammonium hydroxide (TMAH) or by ion-reactive etching with sulfur hexafluoride (SF₆) to obtain the aluminum molding dice 114, 124, as shown in Figs. 16E and 16F. It should be noted that Fig. 16F illustrates the aluminum molding die 124 as flipped over in Fig. 16D.

As illustrated in Fig. 17A, the recess of the aluminum molding die 124 is filled up with melted poly-lactic acid 130 and then the aluminum molding die 114 is inserted into the aluminum molding die 124 as shown in Fig. 17B. Poly-lactic acid 130 is hardened by leaving at room temperature.

Next, phosphoric acid (H₃PO₄) or mixed acid is used to etch the aluminum molding dice 114, 124 to form the tank member 102 and the connector member 103 connecting adjacent tank members 102.

A plurality of anchor members 105 separately prepared according to the third embodiment are adhered onto at least one, preferably all of the bottom surfaces of the tank members 102 with any appropriate biodegradable material, such as glue, starch, protein, and glucose.

Lastly, after any suitable medicaments are fed into each of the tank members 102 through the upper openings 132, all of which in turn are covered and hermetically sealed by a thin layer of poly-lactic acid separately prepared.

### «Drug Delivery System of Sixth Embodiment»

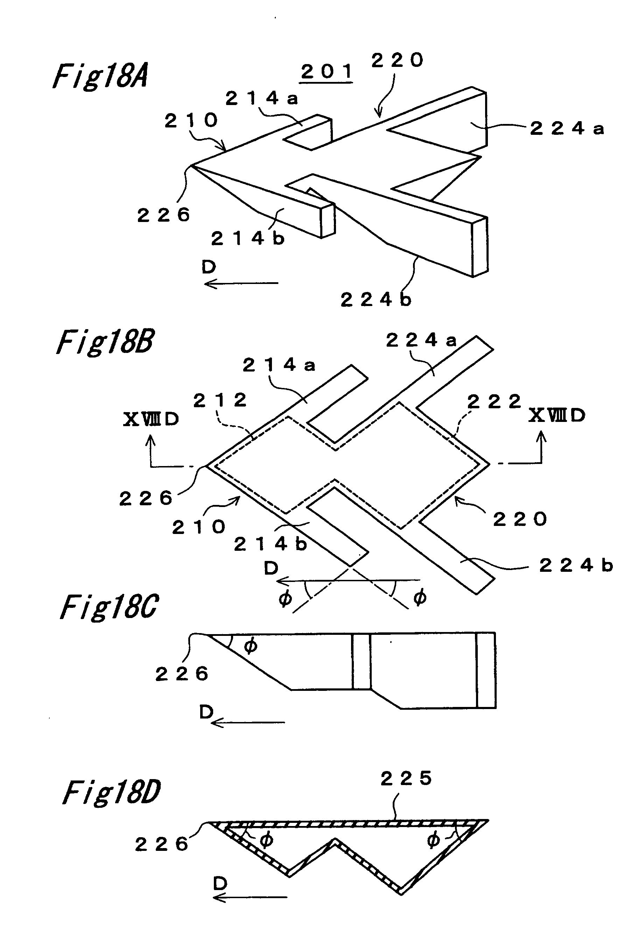

Referring to Figs. 18A-18D, a drug delivery system according to the sixth embodiment will be described herein. In general, the drug delivery system 201 is composed of a plurality (e.g., two) of anchor members 210, 220 combined together, as illustrated in Figs. 18A-18D. The anchor members 210, 220 each have an outline of a prow of a boat and include chambers 212, 222 defined inside, respectively, for holding the medicament such as the regenerative cells and/or factors and the anti-cancer drug. Although the chambers 212, 222 are illustrated as being in fluid communication with each other, they may be designed to be separated. If the chambers 212, 222 are separated, the different type of the medicaments can be preserved in those chambers 212, 222.

Also, although each of the anchor members 210, 220 is illustrated as being a hollow type, i.e., having the chamber therein, it may be a solid type, which is fully filled up with poly-lactic acid. In this case, as described in the fourth embodiment in Fig. 14A, a solid medicine in a tablet form may directly be fixed on one end opposite to the tip 226 of the anchor member 210. Alternatively, as illustrated in Figs. 14B and 14C, a tank member of poly-lactic acid separately prepared may be bonded to the anchor member 220 in an appropriate bonding ways, for example, by using glue or by exposing xenon beam to the local point for heating and melting.

Also, each of the anchor members 210, 220 has a pair of fin-like protruding portions 214a, 214b and 224a, 224b. In the top plan view of the drug delivery system 201 shown in Fig. 18B, each of side lines of the protruding portions 214a, 214b and 224a, 224b is designed such that it is inclined to a longitudinal direction indicated by an arrow D at approximately 35.3 degrees (ϕ = Π/2 - arctan(√2)). Also, in the cross sectional view shown in Fig. 18D, each side line defining the protruding portions 214a, 214b and 224a, 224b of the anchor members 210, 220, except the upper surface 225, is inclined to the longitudinal direction at approximately 35.3 degrees (ϕ = Π/2 - arctan(√2)). Thus, each of the protruding portions 214 and 224a extends rearwardly to the longitudinal direction D, and has a substantially sharp tip 226 in the cross sectional view as well as the top plan view.

In the foregoing description, the drug delivery system 201 has only two anchor members 210 220 combined together, but it may be designed to have only one anchor member or three or more anchor members.

Also, similar to the first embodiment, the anchor members 210, 220 are formed of biodegradable material such as poly-lactic acid, and the sharp tip 226 is formed. Therefore, the drug delivery system 201 can be embedded in a desired portion of a living body where the treatment is required, providing no adverse effect to the body. Also, the anchor members 210, 220 have the protruding portions 214, 224 inclined to the penetration direction (the arrow direction D in Figs. 18A-18D) at an obtuse angle (Π - ϕ). Therefore, once embedded into the treatment portion, the protruding portions 214, 224 engage with the peripheral tissue thereof. This prevents the drug delivery system 201 from being released from the treatment portion and allows it to be secured thereon for a long time even where the treatment portion has a rapid flow of body fluid such as blood. Thus, poly-lactic acid forming the anchor members 210, 220 of the drug delivery system 201 is gently hydrolyzed to dissolve, the medicament held within the chambers 212, 222 can be released in small doses for a predetermined dosing period. Thus, similar to the first embodiment, the drug delivery system 201 is used to form the bypassing blood vessel **BYP** complementing the infarct vessel as shown in Fig. 2B.

### «Manufacturing Process of DDS of Sixth Embodiment»

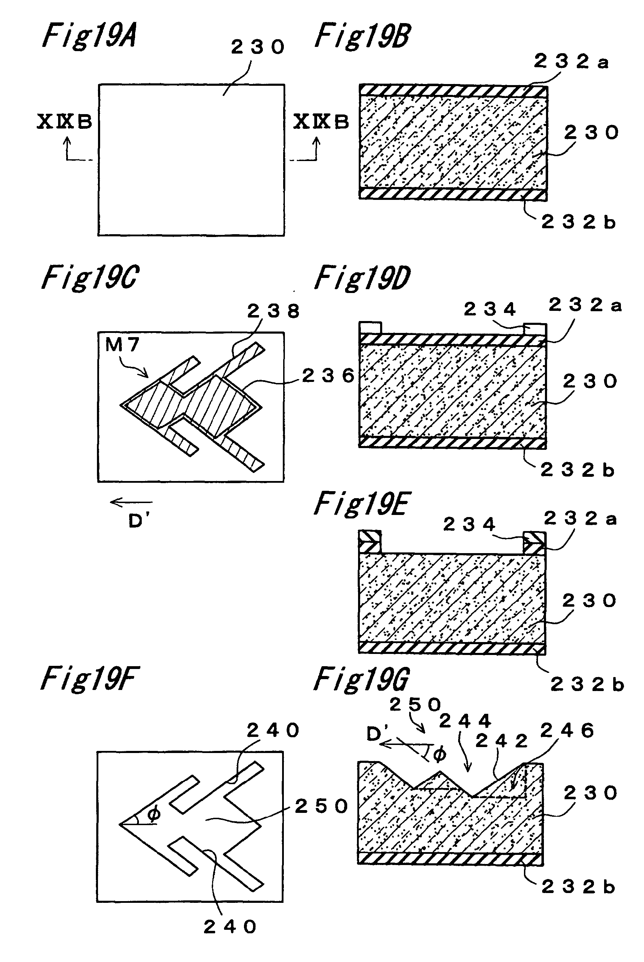

Referring to Figs. 19A-19G, a manufacturing process of the drug delivery system 201 of the sixth embodiment will be described herein.

Contrary to the manufacturing process of the first embodiment, a silicon substrate 230 having principal surfaces of (110) crystal plane is prepared. As shown in Figs. 19A and 19B, the silicon substrates 10 is processed to have silicon dioxide (SiO₂) layers 232a, 232b on both surfaces and washed with sulfuric acid/hydrogen peroxide/water (H₂SO₄ : H₂O₂ = 3 : 1) and ammonium hydroxide/hydrogen peroxide/water (NH₄OH : H₂O₂ : H₂O = 1 : 1 : 5) for five minutes.

A photoresist layer is applied on the silicon dioxide layer and baked at 90 degrees C for ten minutes. Then, a mask M7 shown in Figs. 19C and 19D is used to pattern the photoresist layer 234. This mask M7 does not cover the regions of the photoresist layer 234 indicated by hatchings of Fig. 19C. Thus, the mask M7 uncovers chamber regions 236 and fin regions 238. All of the sides constituting the mask M7 are designed such that they are inclined to the <100> crystal orientation D' of the silicon substrate at approximately 35.3 degrees (ϕ = Π/2 - arctan(√2)).

In Fig. 19E, the silicon dioxide (SiO₂) layer 212a is reactive-ion etched with fluoroform gas (CHF₃) (etching condition: 5sccm, 5Pa, 100W, 1H).

Next, after the photoresist layer 14 is stripped off, the remaining silicon dioxide layer 232a is used as a mask for wet etching the silicon substrate 230 with potassium hydroxide (KOH) as an etchant (etching condition: 33 weight%, 70 degrees C, 55 minutes). As described above, silicon having a surface-orientation dependency (etch anisotropy) with the etchant of potassium hydroxide (KOH) is etched along the orientation perpendicular the (111) crystal plane of silicon. To this result, as shown in Figs. 19F and 19G, the silicon substrate having the principal surface of the (110) crystal plane is etched beneath the mask M7 along the side surface 240 perpendicular to the (110) crystal plane and inclined to the <100> crystal orientation D' of the silicon substrate at approximately 35.3 degrees (ϕ = Π/2 - arctan(√2)), and along the bottom surface 242 inclined to the <100> crystal orientation D' of the silicon substrate at approximately 35.3 degrees (ϕ = Π/2 - arctan(√2)). Thus, a chamber recess 244 is formed in the chamber region 236 as indicated by a solid line, and a fin recess 246 is formed in the fin region 238 as indicated by an imaginary line. The chamber recess 244 and the fin recess 246 together are referred to as an anchor recess 250.

Similar to the manufacturing process of the drug delivery system of the first embodiment (see Figs. 4A-4G), the silicon substrate 230 having the anchor recess 250 is laminated with another silicon substrate separately prepared with silicon dioxide (SiO₂) layers and a thin layer of poly-lactic acid thereon, which is etched with several etchants subsequently. This eventually realizes the drug delivery system 201 of poly-lactic acid. The thickness of the thin layers of poly-lactic acid may be controlled to have each of the chambers 212, 222 to be communicated or separated.

Alternatively, as those skilled in the art can easily conceive, the silicon substrate 230 having the anchor recess 250 is used to obtain the drug delivery system 201 by another manufacturing process similar to one of the second embodiment (see Figs. 10A-10E).

The drug delivery system 201 so formed can safely be placed within a body for a long period so as to release the medicament in small doses.

In the foregoing, the drug delivery system is used for treating the circulatory system disease (forming the bypassing blood vessel). Yet, it can be applied for other diseases. Some of other applications of the drug delivery system will be described herein.

### 1) Regeneration of cornea

When a cornea is damaged by an ophthalmologic disease such as a cataract or glaucoma and/or an accident, the cornea has to be regenerated for treatment. To efficiently regenerating the cornea, the regenerative cells and/or factors for regeneration of the cornea should constantly be supplied to the damaged portion of the cornea. Eye-drops can be used for supplying the regenerative cells and/or factors, however, fresh tears are always circulating in the eye so that most of the regenerative cells and/or factors supplied with eye-drops would immediately run away without staying in the eye. However, according to the drug delivery system of the present invention can be embedded directly into the cornea tissue, so as to constantly and stably supply the regenerative cells and/or factors to the damaged portion of the cornea.

### 2) Treatment of brain disease (Parkinson's disease)

The Parkinson's disease is believed to be developed from the fact that dopamine of the corpus striatum is deficient due to a striatonigral degeneration where dopaminergic neuron cells are degenerated and defected. The medical treatment currently available for the disease is oral dosing of the medicaments including L-dopa agent being modified into dopamine in the corpus striatum, dopamine agonist serving as dopamine, anticholinergic agent improving the balance of dopamine and acetylcholine, and the combination thereof, in order to improve the dopamine deficiency. However, since those medicament orally dosed are diluted in a body, much more amount of the medicament has to be taken into the body. Then, the L-dopa agent causes side effects such as an involontary movement. Thus, the drug delivery system of the present invention is placed within the treatment portion to gently and stably release a desired amount of the medicament only to a portion where the treatment is required, for a long time period.

### 3) Treatment of osseous disease (osteoporosis)

The most effective treatment for the osseous diseases such as the bone fracture and the osteoporosis is believed to directly dose the bone growing factors to the treatment portion. Thus, the drug delivery system according to the present invention can be placed at the treatment portion to supply the bone growing factors in a stable manner.

### 4) Bone growth for orthopaedic and aesthetic plastic surgery

To restore a depressed fracture of a natural skull bone in an accident, an artificial skull bone of plastic material may be implanted. In this instance, the drug delivery system according to the present invention can be used to supply the regenerative factors to the portion of the depressed fracture for regeneration of the natural skull bone. Also, in the aesthetic plastic surgery for extension of a nose, typically, an artificial product such as silicone rubber is implanted into the nose. It is possible to grow the natural nose bone by using the drug delivery system of the invention, thereby to slowly release the regenerative factors for bone growth in small doses for a long time. Advantageously, the nose bone can grow slowly so that no body will recognize the nose bone is extending in such a way.

In the present invention, poly-lactic acid can be used not only for manufacturing the drug delivery system but also other medical devices. Recently, an increase in number of patients having allergenic contact-type dermatitis, in which a skin contacts and rejects metal thereby to irritate, has been reported. It is desirable for those patients to avoid the use of the stainless needle. Although a needle coated with titanium has been proposed, it is quite expensive. According to the present invention, a new needle coated with poly-lactic acid can be provided, which is manufactured by dipping the existing stainless needle into melted poly-lactic acid to form a thin layer of poly-lactic acid on the metal. Therefore, the present invention can be utilized in various medical devices of poly-lactic acid, exploiting the advantage of poly-lactic acid.

## Claims

1. A medical device, comprising:
a tank member of biodegradable material having a chamber capable of holding a medicament; and
at least one anchor member of biodegradable material extending from said tank member;
wherein said anchor member is tapered toward a tip thereof, and has at least one protruding portion extending therefrom.

2. A medical device, comprising:
a plurality of tank members of biodegradable material, each of said tank members having a chamber capable of holding a medicament;
a connector member of biodegradable material connecting adjacent tank members;
a cap member arranged on said connector member for hermetically sealing each of said tank members; and
at least one anchor member of biodegradable material extending from said tank member;
wherein said anchor member is tapered toward a tip thereof, and has at least one protruding portion extending therefrom.

3. A medical device, comprising:
an anchor member of biodegradable material having a chamber capable of holding a medicament;
wherein said anchor member is tapered toward a tip thereof, and has at least one protruding portion extending therefrom.

4. A medical device, comprising:
a tank member of biodegradable material containing a medicament therein; and
at least one anchor member of biodegradable material extending from said tank member;
wherein said anchor member is tapered toward a tip thereof, and has at least one protruding portion extending therefrom.

5. A medical device, comprising:
an anchor member of biodegradable material containing a medicament; and
wherein said anchor member is tapered toward a tip thereof, and has at least one protruding portion extending therefrom.

6. A medical device, comprising:
an anchor member of biodegradable material having a tip tapered at one end in a longitudinal direction, and a mass of a medicament attached at the other end;
wherein said anchor member has at least one protruding portion extending therefrom.

7. A medical device, comprising:
an anchor member of biodegradable material having a chamber capable of sealing a medicament injected therein;
wherein said anchor member has both ends tapered in a longitudinal direction, and has at least one protruding portion extending therefrom.

8. The medical device according to either one of Claims 1 to 7,
wherein the protruding portion of said anchor member has an outline of a substantial quadrangular pyramid.

9. The medical device according to either one of Claims 1 to 7,
wherein the protruding portion extends towards a direction inclined to a longitudinal direction towards the tip at an obtuse angle.

10. The medical device according to either one of Claims 1 to 7,
wherein the biodegradable material is poly-lactic acid, glue, starch, protein, or glucose.

11. The medical device according to Claim 1 or 2,
wherein said anchor member has a channel in fluid communication with the chamber of said tank member.

12. The medical device according to Claim 1 or 2, further comprising a plurality of said anchor members extending from said tank member towards different directions.

13. The medical device according to Claim 1 or 2, further comprising a plurality of said anchor members extending from said tank member towards same directions.

14. The medical device according to Claim 3,
wherein the tip of said anchor member is tapered as viewing in top plan and cross sectional views.

15. A manufacturing process of a medical device, comprising:
forming semiconductor oxide layers on first and second semiconductor substrates;
etching the semiconductor oxide layer on the first semiconductor substrate in a tank region and a plurality of circle regions discretely arranged so as to form a mask of the semiconductor oxide layer;
wet etching the first semiconductor substrate with use of the mask of the semiconductor oxide layer;
forming a semiconductor oxide layer on the first semiconductor substrate exposed by the wet etching;
forming first and second thin layers of poly-lactic acid on the semiconductor oxide layers of the first and second semiconductor substrates, respectively;
laminating the first and second semiconductor substrates so that the first and second thin layers of poly-lactic acid are faced to each other;
etching the first and second semiconductor substrate, while leaving the semiconductor oxide layers of the first and second semiconductor substrates; and
etching the semiconductor oxide layers of the first and second semiconductor substrates, while leaving the first and second thin layers of poly-lactic acid.

16. A manufacturing process of a medical device, comprising:
forming a semiconductor oxide layer on a semiconductor substrate;
etching the semiconductor oxide layer on the semiconductor substrate in a tank region and a plurality of circle regions discretely arranged, except a bridge region extending therethrough so as to form a first mask of the semiconductor oxide layer;
wet etching the semiconductor substrate with use of the first mask of the semiconductor oxide layer;
forming a semiconductor oxide layer on the semiconductor substrate exposed by the wet etching;
forming a thin layer of poly-lactic acid on the semiconductor oxide layer;
forming a thin layer of a given material on the thin layer of poly-lactic acid;
etching the thin layer of the given material in a predetermined region so as to form a second mask of the given material;
etching the thin layer of poly-lactic acid with use of the second mask of the given material;
etching the semiconductor oxide layer with use of the second mask of the given material;
etching the semiconductor substrate, while leaving the semiconductor oxide layer;
etching the thin layer of the given material, while leaving the thin layer of poly-lactic acid; and
etching the semiconductor oxide layer, while leaving the thin layer of poly-lactic acid.

17. A manufacturing process of a medical device, comprising:
forming semiconductor oxide layers on first and second semiconductor substrates;
etching the semiconductor oxide layer on the first semiconductor substrate in a tank region and an anchor region so as to form a mask of the semiconductor oxide layer;
ion-reactive etching the first semiconductor substrate with use of the mask of the semiconductor oxide layer;
forming a semiconductor oxide layer on the first semiconductor substrate exposed by the ion-reactive etching;
forming first and second thin layers of poly-lactic acid on the semiconductor oxide layers of the first and second semiconductor substrates, respectively;
laminating the first and second semiconductor substrates so that the first and second thin layers of poly-lactic acid are faced to each other;
etching the first and second semiconductor substrate, while leaving the semiconductor oxide layers of the first and second semiconductor substrates; and
etching the semiconductor oxide layers of the first and second semiconductor substrates, while leaving the first and second thin layers of poly-lactic acid.

18. A manufacturing process of a medical device, comprising:
forming a semiconductor oxide layer on a semiconductor substrate;
etching the semiconductor oxide layer on the semiconductor substrate in a tank region and an anchor region so as to form a mask of the semiconductor oxide layer;
ion-reactive etching the semiconductor substrate with use of the mask of the semiconductor oxide layer so as to form a recess on the semiconductor substrate in the tank region and the anchor region;
filling up the recess with a given melted material and curing the material so as to form a molding die of the given material;
forming a thin layer of poly-lactic acid encompassing the molding die;
forming an opening on the thin layer of poly-lactic acid to expose a portion of the molding die; and
etching the molding die of the given material, while leaving the thin layer of poly-lactic acid.

19. A manufacturing process of a medical device, comprising:
forming a semiconductor oxide layer on a semiconductor substrate;
etching the semiconductor oxide layer on the semiconductor substrate in an anchor region and a peripheral portion of a tank region so as to form a first mask of the semiconductor oxide layer;
ion-reactive etching the semiconductor substrate with use of the first mask of the semiconductor oxide layer so as to form a recess in the anchor region and the peripheral portion of the tank region;
filling up the recess with a melted poly-lactic acid so as to form a thin layer of poly-lactic acid;
forming a thin layer of a given material on the thin layer of poly-lactic acid;
etching the thin layer of the given material in a predetermined region so as to form a second mask of the given material;
etching the thin layer of poly-lactic acid with use of the second mask of the given material;
etching the semiconductor oxide layer with use of the second mask of the given material;
etching the semiconductor substrate, while leaving the semiconductor oxide layer;
etching the second mask of the given material, while leaving the thin layer of poly-lactic acid;
etching the semiconductor oxide layer, while leaving the thin layer of poly-lactic acid so as to form a structure of poly-lactic acid that includes an opening in a region corresponding to the peripheral portion of the tank region; and
covering the opening of the structure of poly-lactic acid by a thin layer of poly-lactic acid.

20. A manufacturing process of a medical device, comprising:
forming a tank member of poly-lactic acid having a chamber capable of holding a medicament;
forming an anchor member of poly-lactic acid tapered toward to a tip thereof, and said anchor member having at least one protruding portion; and
connecting said anchor member with said tank member.

21. A manufacturing process of a medical device, comprising:
forming first and second recesses on first and second semiconductor substrates, respectively;
filling up the first and second recesses with a given material and curing the material;
etching the first and second semiconductor substrates, while leaving the semiconductor oxide layer so as to form first and second molding dice of the given material;
filling up a die recess of the first molding die with melted poly-lactic acid;
inserting the second molding die into the die recess of the first molding die;
etching first and second molding dice of the given material, while leaving poly-lactic acid therebetween so as to form a plurality of tank members; and
attaching an anchor member to at least one of the tank members.

22. A manufacturing process of a medical device, comprising:
forming first and second semiconductor oxide layers on first and second semiconductor substrates, respectively;
etching the first semiconductor oxide layer on the first semiconductor substrate to form a mask of the first semiconductor oxide layer;
wet etching the first semiconductor substrate with use of the mask of the first semiconductor oxide layer;
forming a semiconductor oxide on the first semiconductor substrate exposed by the wet etching;
forming first and second thin layers of poly-lactic acid on the semiconductor oxide layers of the first and second semiconductor substrates, respectively;
laminating the first and second semiconductor substrates so that the first and second thin layers of poly-lactic acid are faced to each other;
etching the first and second semiconductor substrate, while leaving the semiconductor oxide layers of the first and second semiconductor substrates; and
etching the semiconductor oxide layers of the first and second semiconductor substrates, while leaving the first and second thin layers of poly-lactic acid.

23. A manufacturing process of a medical device, comprising:
forming a semiconductor oxide layer on a semiconductor substrate;
etching the semiconductor oxide layer on the semiconductor substrate in a predetermined mask region so as to form a mask of the semiconductor oxide layer;
wet etching the semiconductor substrate with use of the mask of the semiconductor oxide layer so as to form a recess in the predetermined region;
filling up the recess with a melted give material and curing the material so as to form a molding die of the given material;
forming a thin layer of poly-lactic acid encompassing the molding die;
forming an opening on the thin layer of poly-lactic acid to expose a portion of the molding die; and
etching the molding die of the given material, while leaving the thin layer of poly-lactic acid.

24. The manufacturing process according to Claim 22 or 23,
wherein the mask region is defined by sides inclined to a <100> orientation of the semiconductor substrate at an angle of substantially (Π/2 - arctan(√2)).

25. The manufacturing process according to Claim 16, 19, 21, or 23,
wherein the given material is aluminum.
